(19) Europäisches Patentamt
European Patent Office
Office européen des brevets

(11) **EP 2 789 296 A1**

(12) **EUROPEAN PATENT APPLICATION**
published in accordance with Art. 153(4) EPC

(43) Date of publication:
**15.10.2014 Bulletin 2014/42**

(51) Int Cl.:
**A61B 6/00** *(2006.01)*   **G01T 7/00** *(2006.01)*

(21) Application number: **12855646.1**

(22) Date of filing: **08.11.2012**

(86) International application number:
**PCT/JP2012/079005**

(87) International publication number:
**WO 2013/084658 (13.06.2013 Gazette 2013/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.12.2011  JP 2011266164**
**26.10.2012  JP 2012236818**

(71) Applicant: **Fujifilm Corporation**
**Minato-ku**
**Tokyo 106-0031 (JP)**

(72) Inventors:
• **ISHII Hiroyasu**
**Ashigarakami-gun**
**Kanagawa 258-8538 (JP)**
• **TADA Takuji**
**Ashigarakami-gun**
**Kanagawa 258-8538 (JP)**
• **MURAKOSHI Dai**
**Ashigarakami-gun**
**Kanagawa 258-8538 (JP)**

(74) Representative: **Klunker . Schmitt-Nilson . Hirsch**
**Patentanwälte**
**Destouchesstraße 68**
**80796 München (DE)**

(54) **RADIOGRAPHY APPARATUS**

(57) A radiation photographing apparatus 1 includes a radiation irradiating unit 11, a first grating 31 which has a periodic structure which is formed by arranging a plurality of linear bodies 31b and forms a radiation image including a periodic intensity distribution by the passing radiation; a detecting unit 14 which includes a radiation image detector 30 and acquire radiation image data by detecting the radiation image which is irradiated from the radiation irradiating unit to transmit the first grating and a subject and subjected to modulation for the periodic intensity distribution; and a guide unit 70 which guides the arrangement of the subject in relation to an arrangement direction of a group of the linear bodies of the first grating.

FIG. 11

EP 2 789 296 A1

**Description**

Technical Field

The present invention relates to a radiation photographing apparatus.

Background Art

X-rays are used as a probe for observing an inside of a subject since the X-rays have a characteristic of being attenuated depending on an atomic number of an element which constitutes a material, and a density and a thickness of the material. Photography using X-rays has been widely spread in the fields of medical diagnosis, nondestructive inspection, etc.

In general X-ray photography, a subject is disposed between an X-ray source which emits X-rays and an X-ray image detector which detects an X-ray image so as to photograph a transmission image of the subject. In this case, the X-rays emitted toward the X-ray image detector from the X-ray source suffer from attenuation (absorption) by an amount depending on a difference in characteristics (atomic numbers, densities, and thicknesses) of materials which configure a subject which is present on a path to the X-ray image detector and then, enter the X-ray image detector. As a result, an X-ray transmission image of the subject is detected by the X-ray image detector to be formed as an image. A flat panel detector (FPD), which uses a semiconductor circuit in addition to a combination of an X-ray intensifying screen and a film or a stimulable phosphor (accumulative phosphor), is widely used as an X ray image detector.

However, since a material constituted with an element having a smaller atomic number has a lower X-ray absorption power. Therefore, soft biological tissues, soft materials or the like have a difference in X-ray absorption power therebetween and thus, there is a problem in that light and shade (contrast) sufficient for an X-ray transmission image are not obtained. For example, the component of almost the whole cartilaginous portion which forms a joint of a human body and joint fluid therearound is water and the difference in the X-ray absorption amount therebetween is small so that it is difficult to obtain an image contrast.

Considering such a problem, recently, a study on an X-ray phase imaging has been actively performed in which an image (hereinafter, referred to as a "phase contrast image") based on a phase change (angular change) of the X-ray by the subject is obtained instead of an intensity change of the X-ray by the subject. It is known that when X-rays enter a material, the phase of the X-rays exhibits higher interaction than the intensity of the X-rays. For this reason, the X-ray phase imaging using a phase difference may obtain a high contrast image even from a low absorbing object which has a low X-ray absorbing power, and is useful for visualization of, for example, a cartilaginous portion of a joint such as an interphalangeal joint of a hand or a foot, a cubital joint, or a knee joint.

An X-ray photographing apparatus using an X-ray Talbot interferometer which constituted with two transmission diffraction gratings (a phase grating and an absorption type grating) and an X-ray image detector as a kind of X-ray phase imaging has been designed recently (see, *e.g.,* Patent Literatures 1 and 2).

The X-ray Talbot interferometer is configured by disposing a first diffraction grating (a phase grating or an absorption type grating) behind a subject, disposing a second diffraction grating (an absorption type grating) downstream by a predetermined distance (Talbot interference distance) which is determined by a grating pitch of the first diffraction grating and an X-ray wavelength, and disposing an X-ray image detector behind the second diffraction grating. The Talbot interference distance refers to a distance at which an X-ray which passes through the first diffraction grating forms a self-image (hereinafter, referred to as a "G1 image") representing a periodic intensity distribution by a Talbot interference effect and the G1 image is modulated by an interaction (phase change) between the subject disposed between an X-ray source and the first diffraction grating and the X-rays.

The X-ray Talbot interferometer detects a moire stripe which is generated by superposition of the G1 image and the second diffraction grating, and analyzes the modulation of the periodic pattern which is generated in the image corresponding to the moire stripe by the subject so as to acquire phase information of the subject. For example, a stripe scanning method is known as an analyzing method of the periodic pattern which is generated in the image. According to the stripe scanning method, when the second diffraction grating is photographed plural times while translationally moving the second diffraction grating with respect to the first diffraction grating in a direction which is substantially parallel to a surface of the first diffraction grating and substantially parallel to a pitch direction of the first diffraction grating with a scanning pitch which is obtained by equally dividing a grating pitch of the second diffraction grating, and an angular distribution (a differential image of a phase shift) of the X-rays which are refractive from the subject is acquired from a change in a signal value for every corresponding image between a plurality of obtained image data, it is possible to obtain a phase contrast image of the subject based on the angular distribution.

Citation List

Patent Literature

Patent Literature 1: International Publication WO 08/102598
Patent Literature 2: International Publication WO 08/102685

Summary of Invention

Technical Problem

A differentiation of a phase shift which is acquired by the stripe scanning method is related to the grating pitch direction of the first diffraction grating and edges of the subject which intersect in the grating pitch direction are visualized in the phase contrast image which is obtained based on the differentiation of the phase shift and specifically, edges of the subject which are substantially orthogonal to the grating pitch direction are clearly visualized. That is, the arrangement of the subject is restricted in the grating pitch direction of the first diffraction grating. For example, in order to clearly visualize a cartilaginous portion of a joint in the phase contrast image of a joint such as an interphalangeal joint, a cubital joint, or a knee joint, a finger, an arm or a leg is disposed substantially along the grating pitch direction.

Here, the first and second diffraction gratings are accommodated in an appropriate case for the purpose of protection and dustproof thereof. Further, the first and second diffraction gratings typically need to be configured with a grating pitch in the order of micrometer and with a high aspect ratio and such a grating structure is very fine. Therefore, it is very difficult to directly check the grating pitch direction of the first diffraction grating.

The present invention has been made in consideration of such a situation, and an object of the present invention is to provide a radiation photographing apparatus which enables appropriate disposition of a subject and is capable of obtaining a clear phase contrast image.

Solution to Problem

A radiation photographing apparatus, includes: a radiation irradiating unit; a first grating which has a periodic structure which is formed by arranging a plurality of linear bodies and forms a radiation image including a periodic intensity distribution by passing radiation; a detecting unit which acquires radiation image data by detecting the radiation image which is irradiated from the radiation irradiating unit to transmit the first grating and a subject and subjected to modulation for the periodic intensity distribution; and a guide unit which guides an arrangement of the subject in relation to an arrangement direction of a group of the linear bodies of the first grating.

Advantageous Effects of Invention

According to the present invention, a subject may be appropriately disposed in the relation with an arrangement direction of a linear body group of the first grating and a clear phase contrast image of an interest region of the subject may be obtained.

Brief Description of Drawings

FIG. 1 is a schematic view illustrating a configuration of an example of a radiation photographing apparatus for describing an exemplary embodiment of the present invention.
FIG. 2 is a control block diagram of the radiation photographing apparatus of FIG. 1.
FIG. 3 is a perspective view illustrating a configuration of a photographing unit of the radiation photographing apparatus of FIG. 1.
FIG. 4 is a side view illustrating a configuration of a photographing unit of the radiation photographing apparatus of FIG. 1.
FIG. 5 is a schematic view illustrating a configuration of a radiation image detector included in the photographing unit of FIG. 3.
FIG. 6 is a schematic view for describing refraction of a radioactive ray by a subject.
FIG. 7 is a schematic view for describing an example of a phase contrast image generating method by a stripe scanning method in the radiation photographing apparatus of FIG. 1.

FIG. 8 is a graph illustrating a signal waveform of a pixel of image data which is involved with the stripe scanning.

FIG. 9 is a schematic view for describing another example of a phase contrast image generating method in the radiation photographing apparatus of FIG. 1.

FIG. 10 is a schematic view for describing still another example of a phase contrast image generating method in the radiation photographing apparatus of FIG. 1.

FIG. 11 is a schematic view illustrating an example of an indicator which is marked on a subject table in the radiation photographing apparatus of FIG. 1.

FIG. 12 is a schematic view illustrating another example of an indicator.

FIGS. 13A and 13B are schematic views for illustrating another example of an indicator.

FIG. 14 is a schematic view illustrating a configuration of another example of a radiation photographing apparatus for describing an exemplary embodiment of the present invention.

FIG. 15 is a control block diagram of the radiation photographing apparatus of FIG. 14.

FIGS. 16A and 16B are schematic views for illustrating an example of an indicator which is displayed on a subject table in the radiation photographing apparatus of FIG. 14.

FIG. 17 is a schematic view illustrating a configuration of another example of a radiation photographing apparatus for describing an exemplary embodiment of the present invention.

FIG. 18 is a control block diagram of the radiation photographing apparatus of FIG. 17.

FIGS. 19A and 19B are schematic views for illustrating an example of an arrangement of a subject in the radiation photographing apparatus of FIG. 17.

FIGS. 20A and 20B are schematic views for illustrating another example of an arrangement of a subject in the radiation photographing apparatus of FIG. 17.

FIGS. 21A and 21B are schematic views for illustrating a configuration of a modified embodiment of the radiation photographing apparatus of FIG. 18.

FIG. 22 is a schematic view illustrating a configuration of another example of a radiation photographing apparatus for describing an exemplary embodiment of the present invention.

FIG. 23 is a schematic view illustrating a configuration of another example of a radiation photographing apparatus for describing an exemplary embodiment of the present invention.

FIG. 24 is a schematic view illustrating a configuration of another example of a radiation photographing apparatus for describing an exemplary embodiment of the present invention.

FIG. 25 is a schematic view illustrating a configuration of another example of a radiation photographing apparatus for describing an exemplary embodiment of the present invention.

FIG. 26 is a schematic view illustrating a configuration of another example of a radiation photographing apparatus for describing an exemplary embodiment of the present invention.

Description of Embodiments

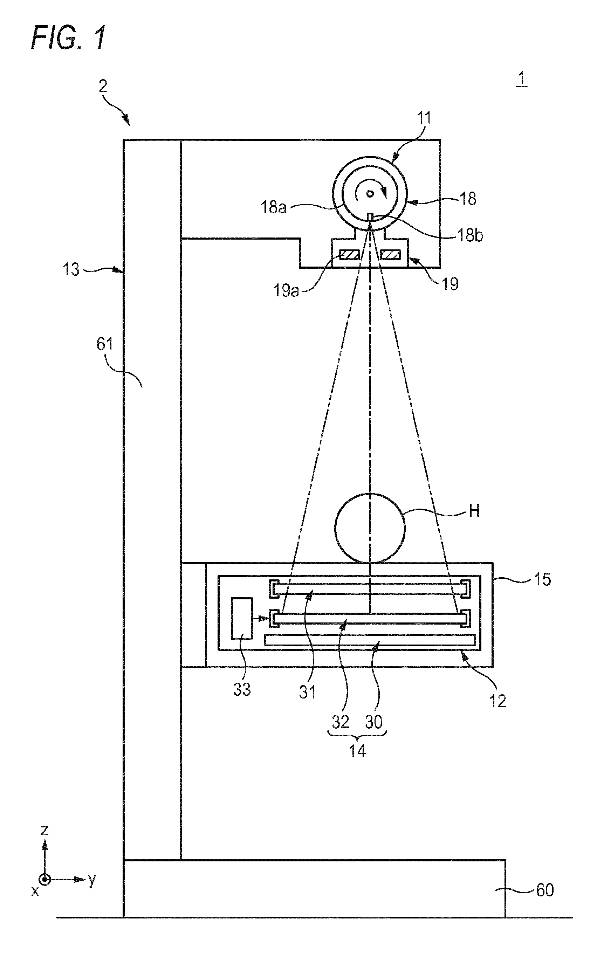

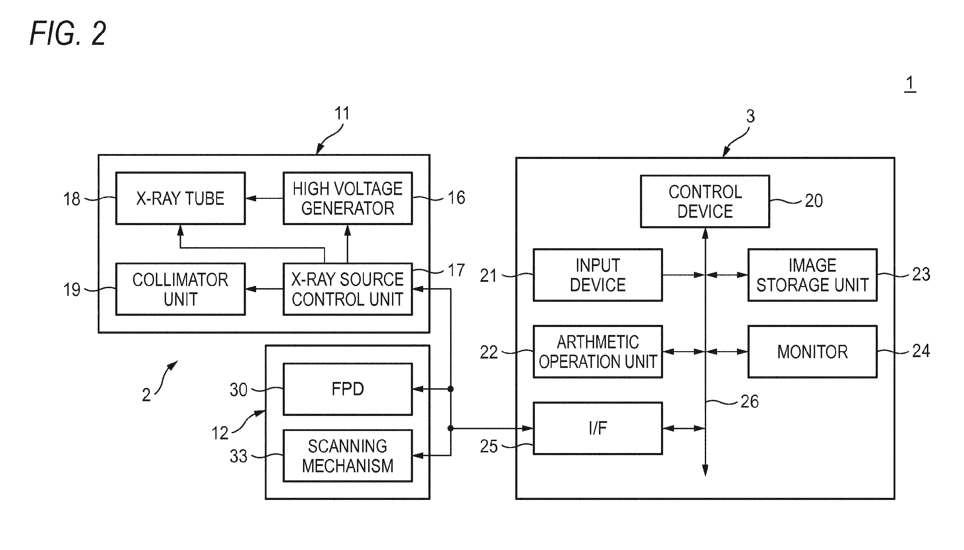

FIG. 1 illustrates a configuration of an example of a radiation photographing apparatus for describing an exemplary embodiment of the present invention, and FIG. 2 is a control block diagram of the radiation photographing apparatus of FIG. 1.

An X-ray photographing apparatus 1 is generally divided into a main body 2 of the X-ray photographing apparatus and a console 3. The main body 2 of the X-ray photographing apparatus includes an X-ray irradiating unit 11 which irradiates an X-ray onto a subject H, a photographing unit 12 which detects the X ray which is radiated from the X-ray irradiating unit 11 to transmit the subject H and generates image data, and a stand 13 which supports the X-ray irradiating unit 11 and the photographing unit 12. The console 3 controls operations of each unit of the main body 2 of the X-ray photographing apparatus, such as an irradiating operation of the X-ray irradiating unit 11 or an photographing operation of the photographing unit 12, based on the manipulation of an operator, and generates a phase contrast image by arithmetically operating the image data acquired by the photographing unit 12.

The stand 13 includes a base 60 which is fixed to a floor and an arm member 61 which extends in a perpendicular direction (a z direction in the illustrated example) from the base 60. The X-ray irradiating unit 11 is attached onto a front end of the arm member 61. A subject table 15 is attached in an approximately central portion of the arm member 61 to face the X-ray irradiating unit 11 in the extension direction of the arm member 61 and the photographing unit 12 is accommodated in the subject table 15.

The X-ray irradiating unit 11 includes an X-ray tube 18 as an X-ray source and a collimator unit 19. The X-ray tube 18 is a rotary anode type and emits an electron beam from a filament (not illustrated) which serves as an electron emission source (cathode) in accordance with a high voltage which is applied from a high voltage generator 16, based on the control of an X-ray source control unit 17, and makes the electron beam collide with a rotary anode 18a which rotates at a predetermined speed to generate an X-ray. A portion of the rotary anode 18a which collides with the electron beam becomes an X-ray focus 18b. The collimator unit 19 includes a movable collimator 19a which limits an irradiation field so as to shield a portion, which does not contribute to an inspection region of the subject H, in the X-ray generated from the X-ray tube 18.

The photographing unit 12 includes a first absorption type grating 31 configured to detect a phase change (an angular change) of the X-ray by the subject H and a detecting unit 14 configured to detect an X-ray image (hereinafter, the X-ray image is referred to as a "G1 image") which is formed by an X-ray which passes through the first absorption type grating 31.

The X-ray photographing apparatus 1 generates a phase contrast image using a stripe scanning method and details thereof will be described below. The detecting unit 14 is provided with a second absorption type grating 32 which is superimposed with the G1 image, an X-ray image detector 30 which detects the G1 image with which the second absorption type grating 32 is superimposed, and a scanning mechanism 33 which translationally moves the second absorption type grating 32 with a predetermined pitch. The scanning mechanism 33 is configured by an actuator such as, for example, a piezoelectric element.

The console 3 is provided with a control device 20 which is configured by a CPU, a ROM, a RAM, or the like. An input device 21 through which the operator inputs a photographing instruction or contents of the instruction, an arithmetic operation processing unit 22 which operates the image data obtained by the photographing unit 12 to generate an X-ray image, an image storage unit 23 which stores the X-ray image, a monitor 24 which displays the X-ray image, and an interface I/F 25 which is connected to individual units of the X-ray photographing apparatus 1 are connected to the control device 20 through buses 26.

A switch, a touch panel, a mouse, or a keyboard may be used as the input device 21 and an X-ray photographing condition such as a voltage of the X-ray tube, an X-ray irradiating time or a photographing timing is input by manipulating the input device 21. The monitor 24 is configured by a liquid crystal display and displays letters such as the X-ray photographing condition or an X-ray image under the control of the control device 20.

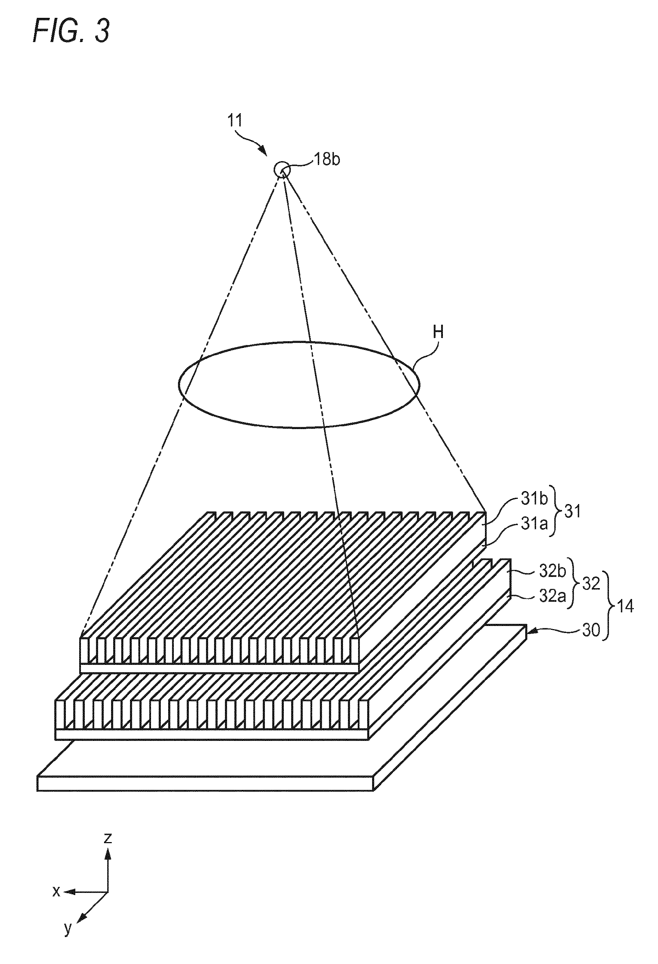

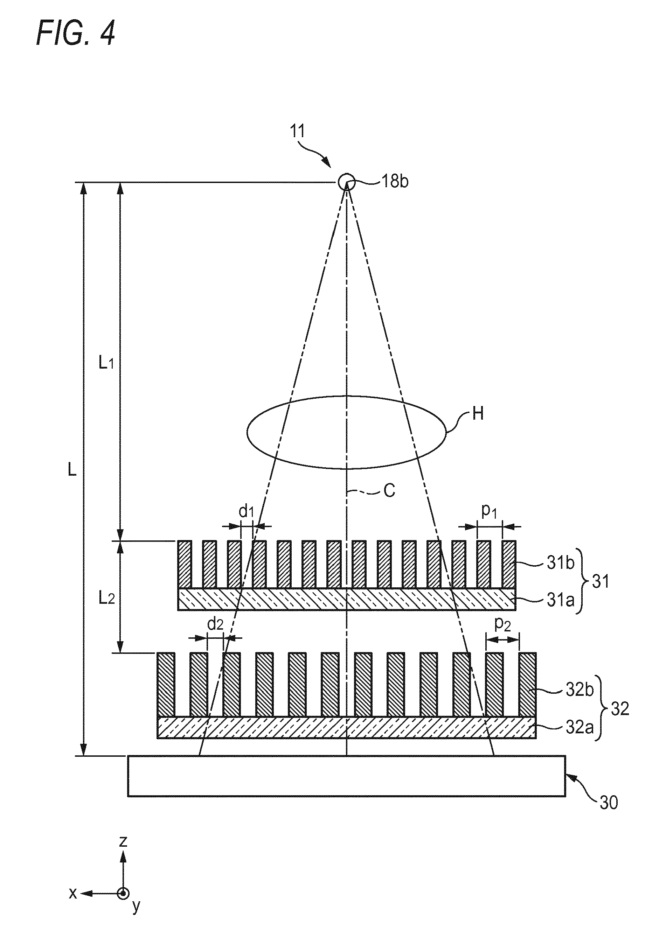

FIGS. 3 and 4 schematically illustrate a configuration of the photographing unit 12.

The first absorption type grating 31 includes a substrate 31a and a plurality of X-ray shielding units 31b (high radiation absorbing units) which is disposed on the substrate 31a. Similarly, the second absorption type grating 32 includes a substrate 32a and a plurality of X-ray shielding units 32b (high radiation absorbing units) which is disposed on the substrate 32a. The substrates 31a and 31b are both formed of an X-ray transmissive member such as silicon, glass, or resin through which the X-ray is transmitted.

The X-ray shield units 31b are configured by linear members, which are elongated in one direction in a plane which is perpendicular to the optical axis C of the X-ray which is radiated from the X-ray irradiating unit 11. As the material for each of the X-ray shielding units 31b, a material, which is excellent in X-ray absorbing property, for example, a heavy metal such as gold or platinum, is desirable. Such X-ray shielding units 31b may be formed by a metal plating method or an evaporation method. The X-ray shielding units 31b are disposed with a predetermined grating pitch $p_1$ and with a predetermined interval $d_1$ in relation to each other in one direction (hereinafter, referred to as an "x direction") which is perpendicular to the direction in the plane which is perpendicular to the optical axis C of the X-ray.

The X-ray shielding units 32b are also configured by linear members which are elongated in one direction in a plane which is perpendicular to the optical axis C of the X-ray which is radiated from the X-ray irradiating unit 11. As the material for each of the X-ray shielding units 32b, a material which is excellent in X-ray absorbing property, for example, a heavy metal such as gold or platinum, is desirable. Such X-ray shielding units 32b may be formed by a metal plating method or an evaporation method. The X-ray shielding units 32b are disposed with a predetermined grating pitch $p_2$ and with a predetermined interval $d_2$ in relation to each other in one direction (the x direction) which is perpendicular to the one direction in the plane which is perpendicular to the optical axis C of the X-ray.

Since the first and second absorption type gratings 31 and 32 configured as described above do not apply a phase difference to the incident X-ray but apply an intensity difference, the first and second absorption type gratings are also referred to as "amplitude type gratings". Further, slit portions (low radiation absorbing portions) which are regions of the intervals $d_1$ and $d_2$ may not be voids and for example, the voids may be filled with a low X-ray-absorbent material such as a polymer or a light metal.

The first and second absorption type gratings 31 and 32 are configured so as to geometrically project the X-rays which pass through the slit portions regardless of existence/non-existence of the Talbot interference effect. Specifically, the intervals $d_1$ and $d_2$ have values which are sufficiently larger than a peak wavelength of the X-ray which is radiated from the X-ray irradiating unit 11 so that most of X-rays which are included in the irradiated X-rays pass through the slit portions while maintaining a straight travelling performance without being diffracted from the slit portions. For example, when tungsten is used as the rotary anode 18a and a tube voltage is 50 kV, the peak wavelength of the X-rays is approximately 0.4 Å. In this case, the intervals $d_1$ and $d_2$ are about 1 to 10 $\mu$m, most of the X-rays are geometrically projected without being diffracted from the slit portions.

The X-rays radiated from the X-ray irradiating unit 11 are not a collimated beam but a corn beam with the X-ray focus 18b as a light emission point so that the G1 image is magnified in proportion to the distance from the X-ray focus 18b. The slit portions of the second absorption type grating 32 are determined to substantially match with a pattern of a periodic intensity distribution of the G1 image in the position of the second absorption type grating 32. That is, when a distance from the X-ray focus 18b to the first absorption type grating 31 is $L_1$ and a distance from the first absorption type grating 31 to the second absorption type grating 32 is $L_2$, the grating pitch $p_2$ is determined to satisfy the relationship of the following Equation 1.

[Equation 1]

$$p_2 = \frac{L_1 + L_2}{L_1} \cdot p_1 \quad \cdots (1)$$

The distance $L_2$ from the first absorption type grating 31 to the second absorption type grating 32 is restricted by a Talbot interference distance which is determined by the grating pitch of the first diffraction grating and the X-ray wavelength, in the Talbot interferometer. However, in the X-ray photographing apparatus 2, the first absorption type grating 31 is configured to project the incident X-rays without diffracting the incident X-rays so that the G 1 image is similarly obtained in all the positions behind the first absorption type grating 31. Therefore, the distance $L_2$ may be set regardless of the Talbot interference distance.

The photographing unit 12 does not configure the Talbot interferometer. However, when it is assumed that the X-rays are diffracted by the first absorption type grating 31, the Talbot interference distance Z is represented by the following Equation 2 using the grating pitch $p_1$ of the first absorption type grating 31, the grating pitch $p_2$ of the second absorption type grating 32, the X-ray wavelength (peak wavelength) $\lambda$, and a positive integer m.

[Equation 2]

$$Z = m \frac{p_1 p_2}{\lambda} \quad \cdots (2)$$

Equation 2 is an equation representing a Talbot interference distance when the X-rays radiated from the X-ray irradiating unit 11 are corn beams and is known by "Atsushi Momose, et al., Japanese Journal of Applied Physics, Vol. 47, No. 10, October of 2008, page 8077".

In the X-ray photographing apparatus 2, the distance $L_2$ is set to be shorter than a minimum Talbot interference distance Z when m = 1, so as to reduce a thickness of the photographing unit 12. That is, the distance $L_2$ is set as a value in a range which satisfies the following Equation 3.

[Equation 3]

$$L_2 < \frac{p_1 p_2}{\lambda} \quad \cdots (3)$$

Further, when it is considered that the X-rays radiated from the X-ray irradiating unit 11 are substantially collimated beams, the Talbot interference distance Z satisfies Equation 4 and the distance $L_2$ is set as a value in a range which satisfies the following Equation 5.

[Equation 4]

$$Z = m \frac{p_1^{\,2}}{\lambda} \quad \cdots (4)$$

[Equation 5]

$$L_2 < \frac{p_1^2}{\lambda} \quad \cdots (5)$$

However, the distance $L_2$ does not necessarily satisfy Equations 3 to 5, for example, when there is no demand for reduction of the thickness of the photographing unit 12, a value in a range departing from Equations 3 to 5 may be employed.

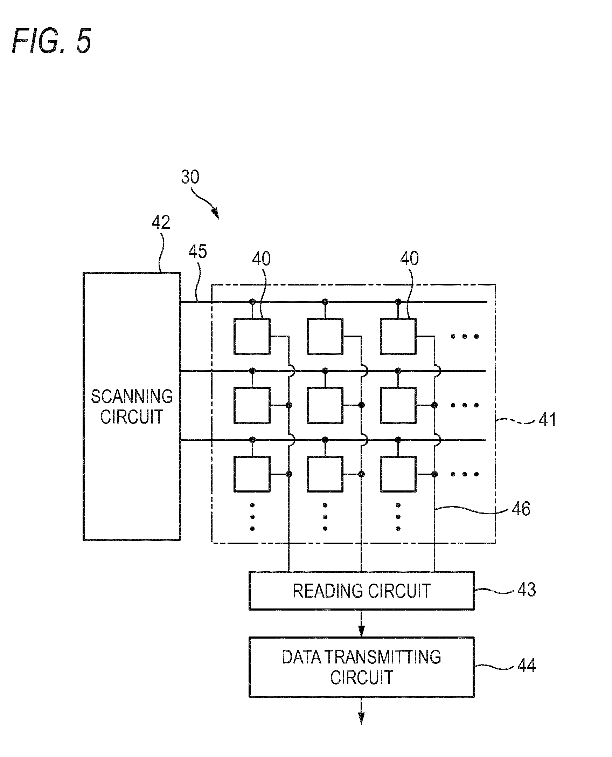

FIG. 5 schematically illustrates a configuration of the X-ray image detector 30.

The X-ray image detector 30 uses a flat panel detector (FPD) which has a thin film transistor (TFT) panel as a base, and includes an image receiving unit 41 in which a plurality of pixels 40 which converts X-rays into electric charges and stores the electric charges is arranged two-dimensionally on a TFT active matrix substrate, a scanning circuit 42 which controls a reading timing of the electric charges from the receiving unit 41, a reading circuit 43 which reads out the electric charges which are stored in each of the pixels 40, converts the electric charge into image data, and stores the image data, and a data transmitting circuit 44 which transmits the image data to the arithmetic operation processing unit 22 through the I/F 25 of the console 3. Further, the scanning circuit 42 and each of the pixels 40 are connected to each other by a scanning line 45 at every row and the reading circuit 43 and each of the pixels 40 are connected to each other by a signal line 46 at every column.

Each of the pixels 40 may be configured as a direct conversion type X-ray detecting element which directly converts an X-ray into an electric charge in a conversion layer (not illustrated) such as amorphous selenium and stores the converted charge in a capacitor (not illustrated) which is connected to a lower electrode. A TFT switch (not illustrated) is connected to each of the pixels 40 in which a gate electrode of the TFT switch is connected to the scanning line 45, a source electrode of the TFT switch is connected to the capacitor, and a drain electrode of the TFT switch is connected to the signal line 46. When the TFT switch is turned ON by a driving pulse from the scanning circuit 42, the electric charges which are stored in the capacitor are read out to the signal line 46.

Each of the pixels 40 may also use a scintillator which converts an X-ray into visible light and may be configured as an indirect conversion type X-ray detecting element which converts the visible light converted in the scintillator into an electric charge and stores the electric charge. As a phosphor which forms the scintillator, for example, terbium activated oxide gadolinium ($Gd_2O_2S$:Tb) or thallium activated cesium iodide (CsI:Tl) is used. Further, the X-ray image detector is not limited to the FPD which has the TFT panel as a base but various X-ray image detectors which use a solid state imaging device such as a CCD sensor or a CMOS sensor as a base may be used.

The reading circuit 43 is configured by an integral amplifier circuit, an A/D converter, a correcting circuit, and an image memory. The integral amplifier circuit integrates the electric charge which is output from each of the pixels 40 through the signal line 46 and converts the electric charge into a voltage signal (an image signal) to input the converted signal to the A/D converter. The A/D converter converts the input image signal into digital image data and inputs the converted digital image data to the correcting circuit. The correcting circuit performs offset correction, gain correction, and linearity correction on the image data and stores the corrected image data in the image memory. Further, the correction processing by the correcting circuit may include correction of an amount of exposed X-rays or an exposure distribution (so called shading), or correction of a pattern noise (for example, a leak signal of the TFT switch) which depends on a control condition (a driving frequency or a reading period) of the X-ray image detector 30.

The X-ray image detector 30 is disposed such that the image receiving surface thereof is perpendicular to the optical axis C of the X-rays and a row direction or a column direction in the arrangement of the pixels 40 of the receiving unit 41 is typically parallel to the grating pitch direction (x direction) of the first absorption type grating 31.

In the photographing unit 12 configured as described above, the G1 image which represents a periodic intensity distribution to which the grating structure of the first absorption type grating 31 is reflected by the X-rays which pass through the first absorption type grating 31 is formed. The G1 image is intensity-modulated through superimposition with the second absorption type grating 32 and the intensity-modulated G1 image modulated is captured by the X-ray image detector 30. In the position of the second absorption type grating 32, a pattern period $p_1$' of the periodic intensity distribution of the G1 image and a substantial grating pitch $p_2$' (an actual pitch after being manufactured) of the second absorption type grating 32 may slightly vary due to a manufacturing error or an arrangement error. Among them, the arrangement error means that inclination, rotation or a distance between the first and second absorption type gratings 31 and 32 is relatively changed so that a substantial pitch in the x direction is changed.

By a minute difference between the pattern period $p_1$' of the G1 image and the grating pitch $p_2$' of the second absorption type grating 32, an image contrast on the X-ray image detector 30 includes moire stripes. A period T of the moire stripes in the x direction is represented by the following Equation 6 in which a distance from the X-ray focus 18b to the X-ray image detector 30 is L.

[Equation 6]

$$T = \frac{L}{L_1 + L_2} \left| \frac{p_1' \times p_2'}{p_1' - p_2'} \right| \quad \cdots (6)$$

In order to detect the moire stripes from the X-ray image detector 30, it is required that an arrangement pitch P of the pixel 40 in the x direction is not at least integral multiple of the moire period T and the arrangement pitch P satisfies the following Equation 7 (here, n is a positive integer).

[Equation 7]

$$P \neq nT \quad \cdots (7)$$

Further, in a range which satisfies Equation 7, even when the arrangement pitch P is larger than the moire period T, the moire stripes may be detected. However, it is desirable that the arrangement pitch P is smaller than the moire period T and it is desirable that the arrangement pitch P satisfies the following Equation 8. This is because, in order to obtain a good phase contrast image, it is desirable that the moire stripes are detected at a high contrast in a process of generating a phase contrast image which will be described below.

[Equation 8]

$$P < T \quad \cdots (8)$$

The arrangement pitch P of the pixels 40 has a value (generally, approximately 100 $\mu$m) which is determined in accordance with a design so that it is difficult to change the arrangement pitch P. Therefore, in order to adjust a size relationship between the arrangement pitch P of the pixels 40 and the period T of the moire stripes, it is desirable to change the period T of the moire stripe by adjusting the positions of the first and second absorption type gratings 31 and 32 and changing at least one of the pattern period $p_1'$ of the G1 image and the grating pitch $p_2'$ of the second absorption type grating 32. The period T of the moire stripes may be changed, for example, by relatively rotating the first and second absorption type gratings 31 and 32 with respect to the optical axis C, relatively moving the first and second absorption type gratings 31 and 32 along the optical axis C, or relatively slanting the first and second absorption type gratings 31 and 32.

When the subject H is disposed on the subject table 15, the periodic intensity distribution of the G1 image is subjected to modulation by the subject H and the moire stripes by the superimposition of the G1 image and the second absorption type grating 32 are also subjected to modulation. The modulated amount is proportional to an angle of the deflected X-ray due to the refraction effect by the subject H. The image acquired by the X-ray image detector 30 includes a periodic pattern corresponding to the moire stripes and the periodic pattern is analyzed to generate a phase contrast image of the subject H.

Hereinafter, an analyzing method of a periodic pattern of an image will be described.

[Analyzing Method 1]

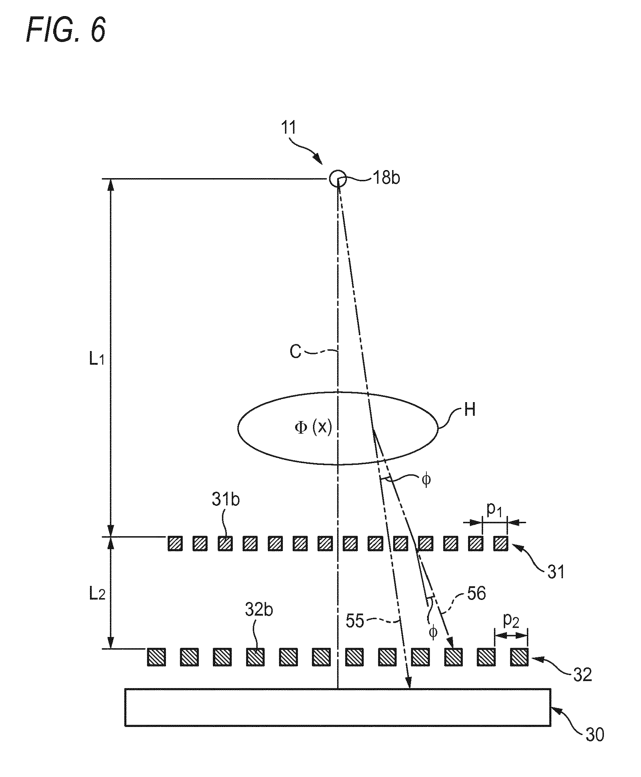

FIG. 6 illustrates one X-ray which is refracted along a phase shift distribution $\Phi(x)$ of the subject H in the x direction.

Reference numeral 55 denotes a path of an X-ray which goes straight when no subject H is present, and the X ray which travels along the path 55 passes through the first and second absorption type gratings 31 and 32 to be incident onto the X-ray image detector 30. Reference numeral 56 denotes a path of an X-ray which is refracted and deflected by the subject H when the subject H is present. The X-ray which travels along the path 56 passes through the first absorption type grating 31 and then is shielded by the second absorption type grating 32.

When a refractive index distribution of the subject H is n(x, z) and z is a direction where the X-ray travels, the phase shift distribution $\Phi(x)$ of the subject H is represented by the following Equation 9.

[Equation 9]

8

$$\Phi(x) = \frac{2\pi}{\lambda} \int [1 - n(x,z)] dz \quad \cdots (9)$$

The G1 image which is projected from the first absorption type grating 31 onto the position of the second absorption type grating 32 is displaced in the x direction by an amount corresponding to a refraction angle φ by the refraction of the X-ray from the subject H. The displacement amount Δx is approximately represented by the following Equation 10 based on a fact that the refraction angle φ of the X-ray is minute.
[Equation 10]

$$\Delta x \approx L_2 \phi \quad \cdots (10)$$

Here, the refraction angle φ is represented by Equation 11 using the X-ray wavelength λ and the phase shift distribution Φ(x) of the subject H.
[Equation 11]

$$\phi = \frac{\lambda}{2\pi} \frac{\partial \Phi(x)}{\partial x} \quad \cdots (11)$$

As described above, the displacement amount Δx of the G1 image by the refraction of the X-ray from the subject H is related with the phase shift distribution Φ(x) of the subject H. Further, the displacement amount Δx is related with a phase deviation amount ψ (a phase difference of the signals between a case when the subject H is present and a case when no subject H is present) of a signal of each pixel of the image data, as represented in the following Equation 12.
[Equation 12]

$$\phi = \frac{2\pi}{p_2} \Delta x = \frac{2\pi}{p_2} L_2 \phi \quad \cdots (12)$$

Therefore, when the phase deviation amount ψ of the signal of each pixel is obtained, the refraction angle φ is obtained from Equation 12, and a differential amount of the phase shift distribution Φ(x) is obtained using Equation 11. Therefore, the differential amount is integrated with respect to x to generate the phase shift distribution Φ(x) of the subject H, that is, a phase contrast image of the subject H. In the X-ray photographing apparatus 1, the phase deviation amount ψ is calculated using the stripe scanning method which will be described below.

According to the stripe scanning method, photographing is performed while moving one of the first and second absorption type gratings 31 and 32 with respect to the other grating in a grating pitch direction of the one grating stepwise in a translation manner and changing a phase of the periodic arrangement of the X-ray shielding unit 32b of the second absorption type grating 32 with respect to the periodic intensity distribution of the G1 image. In the present X-ray photographing apparatus 2, the second absorption type grating 32 is moved by the scanning mechanism 33 (see FIG. 1), but the first absorption type grating 31 may be moved.

In accordance with the movement of the second absorption type grating 32, when the moire stripes are moved and the translation distance reaches one period (grating pitch $p_2$) of the grating period of the second absorption type grating 32 (that is, the phase change reaches 2π), the moire stripes return to their original position. Such a change of the moire stripes is imaged by the X-ray image detector 30 while moving the second absorption type grating 32 by one N-th (N is an integer) of the grating pitch $p_2$ and a plurality of signal values per every pixel is obtained from a plurality of obtained image data to be operated in the arithmetic operation processing unit 22 so that a phase deviation amount ψ of the signal of each pixel is obtained.

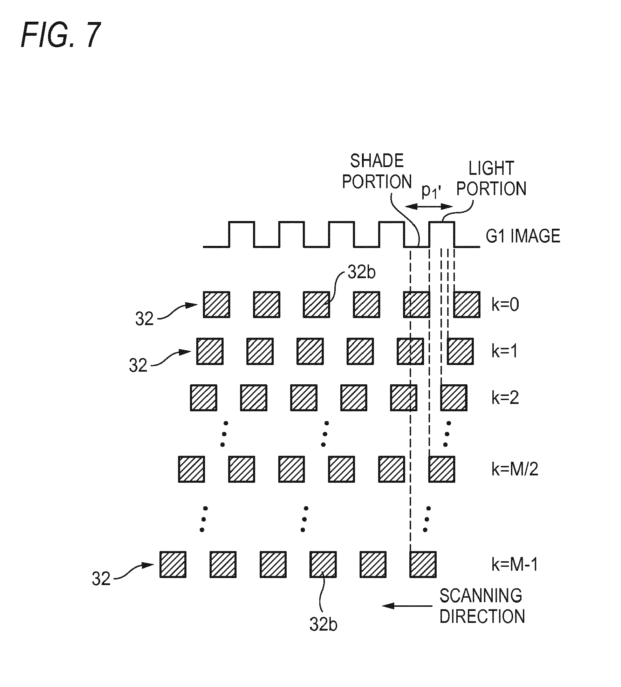

FIG. 7 schematically illustrates a state when the second absorption type grating 32 is moved by a scanning pitch ($p_2$/M) obtained by dividing the grating pitch $p_2$ into M (2 or larger integer).

The scanning mechanism 33 sequentially and translationally moves the second absorption type gratings 32 to M scanning positions (k = 0, 1, 2, ..., M-1). Further, in FIG. 7, the initial position of the second absorption type grating 32 is set to a position (k = 0) where a shade portion of the G1 image at the position of the second absorption type grating 32 when no subject H is present approximately matches to the X-ray shielding unit 32b. However, the initial position may be set to any position of k = 0, 1, 2, ..., M-1.

First, at the position of k = 0, the X-ray which is not refracted by the subject H mainly passes through the second absorption type grating 32. Next, when the second absorption type grating 32 moves to k = 1, 2, ..., in sequence, the X-ray which passes through the second absorption type grating 32 is subjected to the decrease of components of the X-ray which are not refracted by the subject H and the increase of components of the X-ray which are refracted by the subject H. Specifically, when k = M/2, only the X-ray which is refracted by the subject H mainly passes through the second absorption type grating 32. When k exceeds M/2, in contrast, the X-ray which passes through the second absorption type grating 32 is subjected to the reduction of the components of the X-ray which are refracted by the subject H but the increase of the components of the X-ray which are not refracted. When imaging is performed by the X-ray image detector 30 at each of the positions of k = 0, 1, 2, ... M-1, M signal values for each pixel may be obtained. Hereinafter, a method of calculating a phase deviation amount $\psi$ of the signal of each of the pixels from M signal values will be described.

Assuming that a signal value of each pixel when the second absorption type grating 32 is at a position k is $I_k(x)$, $I_k(x)$ is represented by Equation 13.

[Equation 13]

$$I_k(x) = A_0 + \sum_{n>0} A_n \exp\left[2\pi i \frac{n}{p_2}\left\{L_2\phi(x) + \frac{kp_2}{M}\right\}\right] \quad \cdots(13)$$

Here, x is a coordinate of each of the pixels in the x direction, $A_0$ is an intensity of an incident X-ray, and $A_n$ is a value corresponding to a contrast of the signal (here, n is a positive integer). Further, $\Phi(x)$ represents a refraction angle $\varphi$ as a function of a coordinate x of the pixel.

Next, upon using the following relation equation, Equation 14, the refraction angle $\varphi(x)$ may be represented by the following Equation 15.

[Equation 14]

$$\sum_{k=0}^{M-1} \exp\left(-2\pi i \frac{k}{M}\right) = 0 \quad \cdots(14)$$

[Equation 15]

$$\phi(x) = \frac{p_2}{2\pi L_2} \arg\left[\sum_{k=0}^{M-1} I_k(x) \exp\left(-2\pi i \frac{k}{M}\right)\right] \quad \cdots(15)$$

Here, arg[ ] indicates extraction of a deflection angle and corresponds to a phase deviation amount $\psi$ of the signal of each pixel. Therefore, the phase deviation amount $\psi$ of the signal of each pixel is calculated from M signal values which are obtained for each pixel based on Equation 15 so that the refraction angle $\varphi(x)$ is obtained.

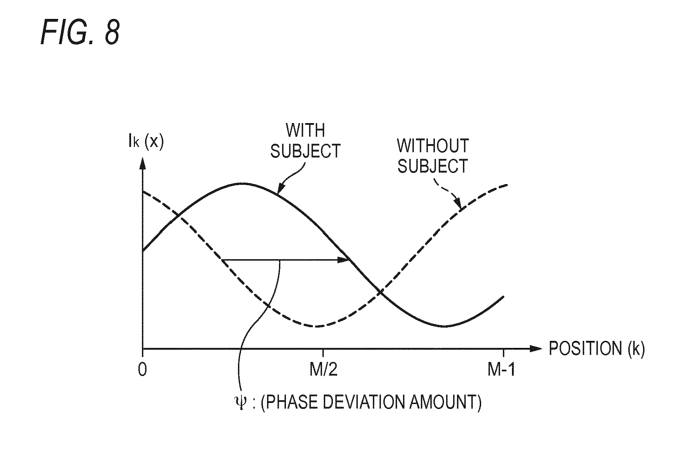

FIG. 8 illustrates a signal waveform of one pixel which is changed in accordance with the stripe scanning.

The M signal values obtained for each pixel are periodically changed with respect to the position k of the second absorption type grating 32 with a period of the grating pitch $p_2$. The broken line of FIG. 8 indicates a signal waveform when no subject H is present and the solid line of FIG. 8 indicates a signal waveform when a subject H is present. The phase difference of both waveforms corresponds to the phase deviation amount $\psi$ of the signal of each pixel.

The refraction angle $\varphi(x)$ is integrated along the x axis so as to correspond the differentiation of the phase shift distribution $\Phi(x)$ as represented in Equation 11, so that the phase shift distribution $\Phi(x)$ is obtained. Further, in the above description, a y coordinate in a y direction of the pixel is not considered, but the same operation is performed on the y coordinate so that two dimensional phase shift distribution $\Phi(x, y)$ in the x direction and the y direction is obtained. The above-mentioned arithmetic operation is performed by the arithmetic operation processing unit 22 and the arithmetic operation processing unit 22 stores the phase shift distribution $\Phi(x, y)$ in the image storage unit 23 as a phase contrast image.

[Analyzing Method 2]

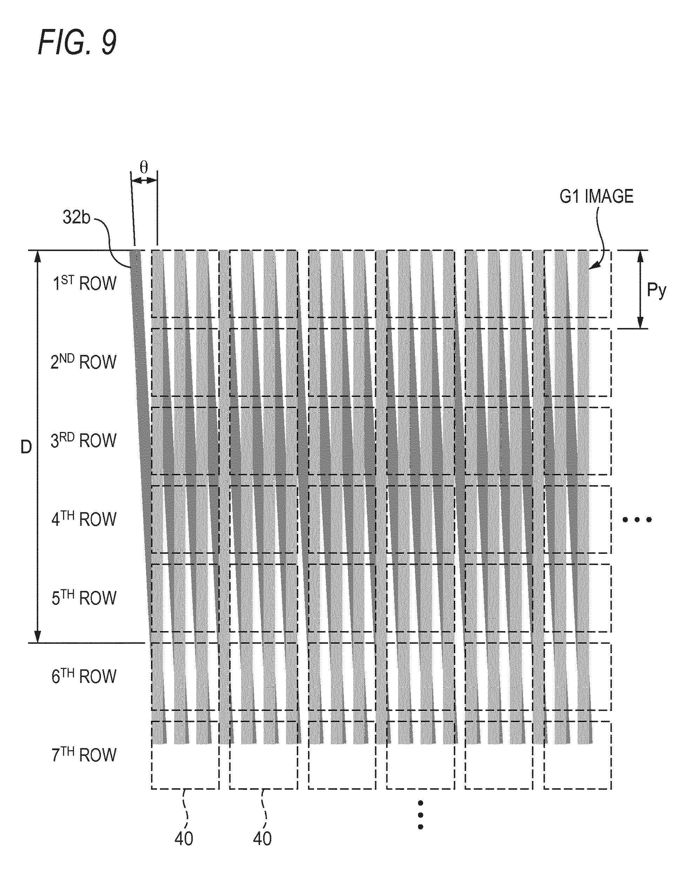

FIG. 9 illustrates another example of a phase contrast image generating method in the X-ray photographing apparatus 1.

In the method which will be described below, a plurality of image data which is equivalent to the plurality of image data acquired by the above-described stripe scanning method is acquired by photographing one time.

When the first and second absorption type gratings 31 and 32 are disposed to be relatively rotated by an angle θ around the optical axis C, the G1 image and the second absorption type grating 32 are relatively rotated by the angle θ. The superimposition of the periodic intensity distribution of the G1 image at a position on an image receiving surface of the X-ray image detector 30 and the projection (projection of the periodic arrangement of the X-ray shielding units 32b) of the second absorption type grating 32 is periodically changed in a direction (y direction) perpendicular to the grating pitch direction (the x direction) of the first absorption type grating 31.

The rotation angle θ is set such that the arrangement pitch of the pixel 40 in the y direction is Py, the number of image data to be obtained is M, and a distance D corresponding to an n period (but, n is an integer except 0 and multiples of M) of the change is D = Py X M. Accordingly, with M pixels 40 which are adjacent in the y direction as one unit, phases of the periodic intensity distribution of the G1 image and the periodic arrangement of the X-ray shielding units 32b of the second absorption type grating 32 are different from each other between the M pixels for each unit. The illustrated example represents a case in which M = 5 and n = 1.

With a plurality of pixel rows with an interval of M rows as one set, image data is formed based on an electric charge which is read out from each of the pixels 40 of the pixel row group of the set, for every set so that M pieces of image data are obtained. As illustrated in the drawing, when the rotation angle θ is set under the condition that M = 5 and n = 1, first image data is obtained from a pixel row group of (5Xk-4) rows (k = 1, 2, ...), second image data is obtained from a pixel row group of (5Xk-3) rows, third image data is obtained from a pixel row group of (5Xk-2) rows, fourth image data is obtained from a pixel row group of (5Xk-1) rows, and fifth image data is obtained from a pixel row group of (5Xk) rows. A method of generating a phase contrast image based on M image data which are obtained as described above is the same as the above-described stripe scanning method.

According to the method of generating a phase contrast image, a plurality of images required to analyze the periodic pattern is obtained by photographing one time and the method does not require the movement of the first absorption type grating 31 or the second absorption type grating 32 which is required during the photographing of plural times and the scanning mechanism 33 which requires high precision. Therefore, it is possible to improve a photographing work flow and simplify the apparatus. Further, it is possible to prevent an image quality from being degraded due to the movement of the subject from photographing to photographing.

[Analyzing Method 3]

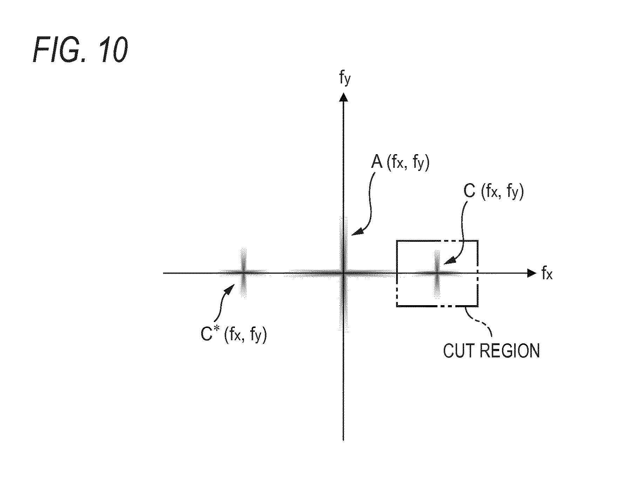

FIG. 10 illustrates another example of a phase contrast image generating method in the X-ray photographing apparatus 1.

In the method which will be described below, instead of the above-described stripe scanning method, Fourier transformation and inverse Fourier transformation are used to analyze the periodic pattern of the image and generate a phase contrast image.

The periodic pattern of the image corresponding to the moire stripes which are formed by the superimposition of the G1 image and the second absorption type grating 32 is represented by the following Equation 16 and Equation 16 may be rewritten as the following Equation 17.

[Equation 16]

$$f(x,y) = a(x,y) + b(x,y)\cos(2\pi(f_{0x}x + f_{0y}y) + \phi(x,y)) \quad \cdots(16)$$

[Equation 17]

$$f(x,y) = a(x,y) + c(x,y)\exp(2\pi i(f_{0x}x + f_{0y}y)) + c^{*}(x,y)\exp(-2\pi i(f_{0x}x + f_{0y}y)) \quad \cdots(17)$$

In Equation 16, a(x, y) indicates a background, b(x, y) indicates an amplitude of a spatial frequency component corresponding to the basic period of the periodic pattern, and $(f_{0x}, f_{0y})$ indicates a basic period of the periodic pattern. Further, in Equation 17, c(x, y) is represented by the following Equation 18.
[Equation 18]

$$c(x, y) = \frac{1}{2} b(x, y) \exp[i\,\phi(x, y)] \quad \cdots (18)$$

Therefore, when a component of c(x, y) or c*(x, y) is extracted, information on the refraction angle φ(x, y) may be obtained. Here, Equation 17 becomes Equation 19 by Fourier transformation.
[Equation 19]

$$F(f_x, f_y) = \mathrm{A}(f_x, f_y) + \mathrm{C}(f_x - f_{0x}, f_y - f_{0y}) + \mathrm{C}^*(f_x + f_{0x}, f_y + f_{0y}) \quad \cdots (19)$$

In Equation 19, $F(f_x, f_y)$, $A(f_x, f_y)$, and $C(f_x, f_y)$ are two-dimensional Fourier transformations of f(x, y), a(x, y), and c(x, y), respectively.
When one-dimensional gratings such as the first and second absorption type gratings 31 and 32 are used, at least three types of peaks including a peak derived from $A(f_x, f_y)$ and peaks of spatial frequency components corresponding to a basic period of the periodic patterns derived from $C(f_x, f_y)$ and $C^*(f_x, f_y)$ with $A(f_x, f_y)$ therebetween are generated in the spatial frequency spectrum of the image, as illustrated in FIG. 10. The peak derived from $A(f_x, f_y)$ is generated at an original point, and the peaks derived from $C(f_x, f_y)$ and $C^*(f_x, f_y)$ are generated at the positions of $(\pm f_{0x}, \pm f_{0y})$ (double signs in same order).
In order to obtain the refraction angle φ(x, y) from the spatial frequency spectrum of the image, a region which includes a peak frequency of the spatial frequency component corresponding to the basic period of the period pattern is cut out and the cut region is moved so that the peak frequency is superimposed with the original point of the frequency space to perform the inverse Fourier transformation. Further, the refraction angle φ(x, y) may be obtained from complex information which is obtained by the inverse Fourier transformation. A method of generating a phase contrast image from the refraction angle φ(x, y) is the same as the above-described stripe scanning method.
According to the method of generating a phase contrast image described above, the phase contrast image is generated from one periodic pattern image and thus, the method ends by photographing one time so that the method does not require the movement of the first absorption type grating 31 or the second absorption type grating 32 which is required during the photographing of plural times and the scanning mechanism 33 which requires high precision. Therefore, it is possible to improve a photographing work flow and simplify the apparatus. Further, it is possible to prevent an image quality from being degraded due to the movement of the subject from photographing to photographing.
In Analyzing Methods 1 to 3 of a periodic pattern of the image which have been described above, the refraction angle φ of the first absorption type grating 31 in the x direction which is the grating pitch direction, that is, differentiation of the phase shift distribution Φ is obtained and an edge of the subject H which intersects the grating pitch direction of the first absorption type grating 31 is visualized in the phase contrast image which is obtained based on the differentiation of the phase shift distribution Φ. Specifically, the edge of the subject H which is approximately perpendicular to the grating pitch direction of the first absorption type grating 31 is clearly visualized. That is, the arrangement of the subject H is restricted by the grating pitch direction of the first absorption type grating 31. Therefore, in the X-ray photographing apparatus 1, in relation to the grating pitch direction of the first absorption type grating 31, a guide unit which guides the arrangement of the subject H on the subject table 15 is provided.

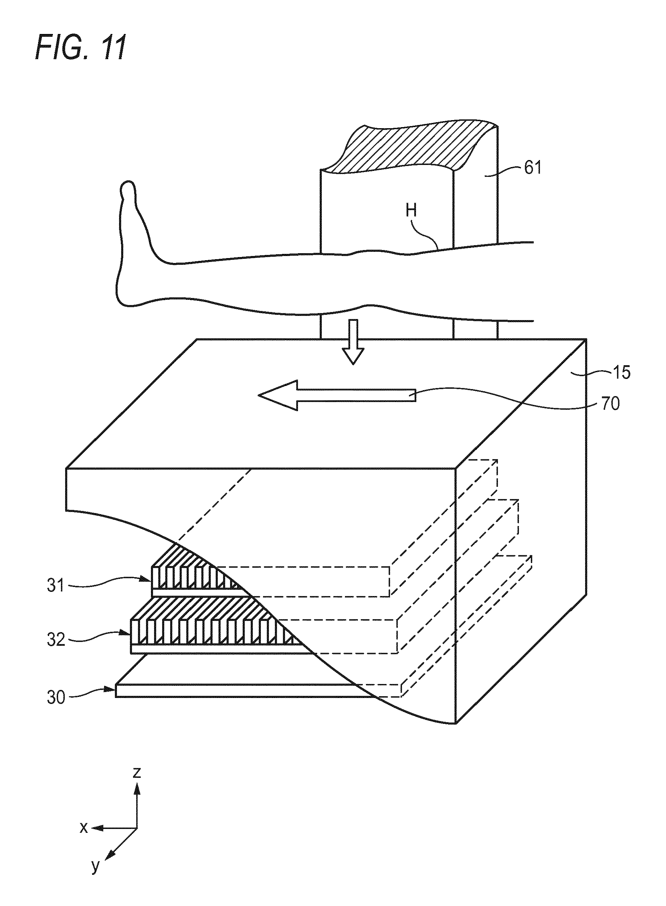

FIG. 11 illustrates an example of the guide unit.
In the example illustrated in FIG. 11, an arrow 70 which extends in the grating pitch direction (the x direction) of the first absorption type grating 31 is marked on a disposing surface of the subject table 15, and the grating pitch direction of the first absorption type grating 31 is indicated by this indicator.
For example, when the subject H is a knee joint and the cartilaginous portion is a region of interest, the knee joint is disposed such that a thighbone and a shinbone which form the knee joint extend in the indicated direction of the arrow 70 which is the indicator. Therefore, an edge of the cartilaginous portion which is a region of interest is disposed to be approximately perpendicular to the grating pitch direction of the first absorption type grating 31 and clearly visualized in the phase contrast image.

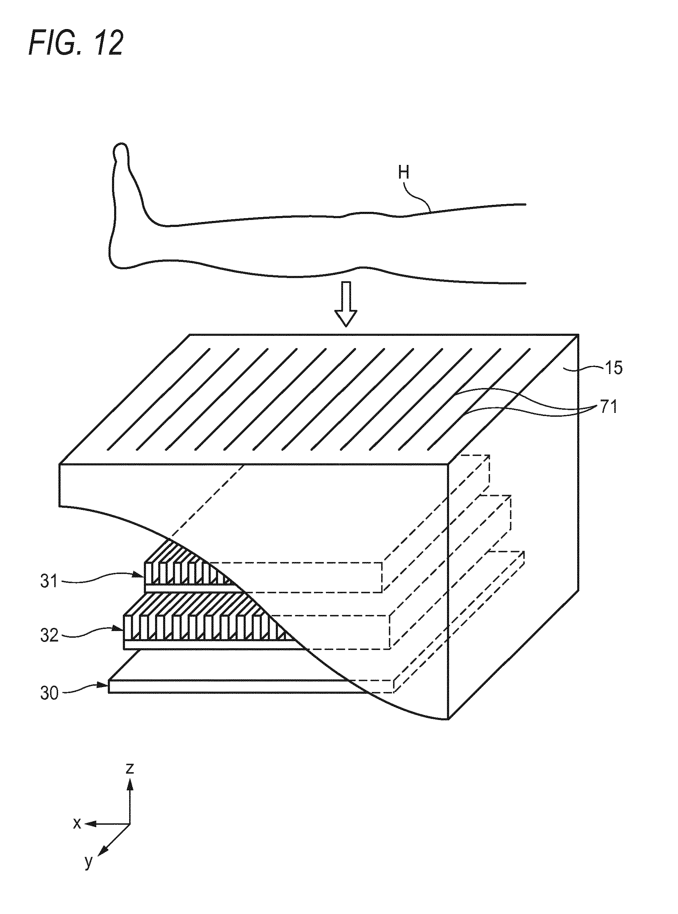

FIG. 12 illustrates another example of the guide unit.

In the example illustrated in FIG. 12, a plurality of lines 71 which extends to be parallel to the X-ray shielding unit 31b of the first absorption type grating 31 and is arranged in the grating pitch direction is marked on the disposing surface of the subject table 15 and the extending direction of the X-ray shielding unit 31b of the first absorption type grating 31 is visualized by this indicator.

For example, when the subject H is a knee joint of a leg and the cartilaginous portion is a region of interest, the knee joint is disposed such that a thighbone and a shinbone which form the knee joint are substantially perpendicular to the plurality of lines which is indicators so that an edge of the cartilaginous portion which is a region of interest is disposed to be approximately perpendicular to the grating pitch direction of the first absorption type grating 31 and clearly visualized in the phase contrast image.

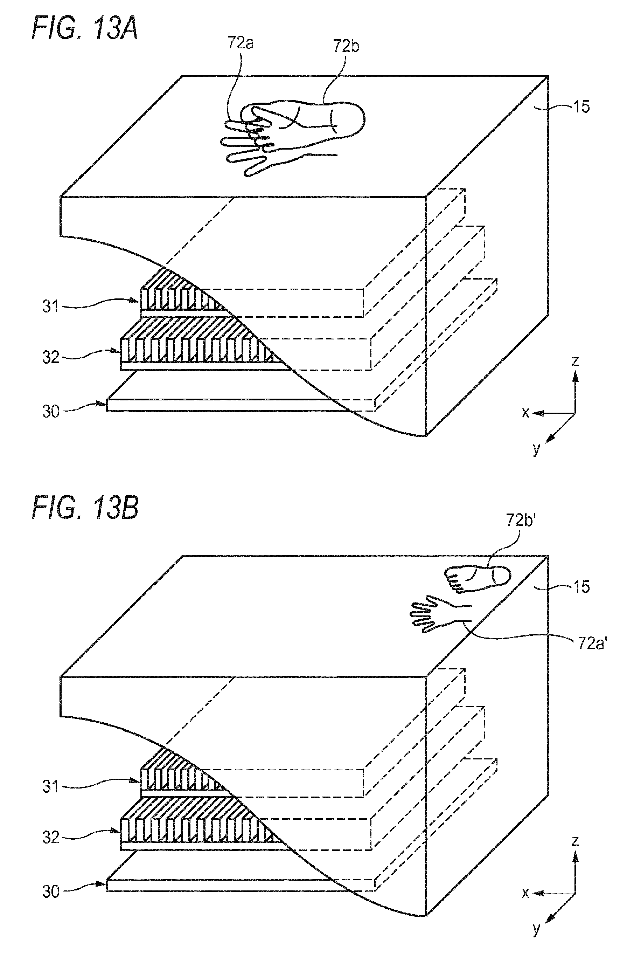

FIGS. 13A and 13B illustrate another example of the guide unit.

In the example illustrated in FIGS. 13A and 13B, a figure representing a sketch of the subject is marked on the disposing surface of the subject table 15 for every type of subject. In an example that the type of subject is an interphalangeal joints of a hand and an interphalangeal joints of a foot, a figure 72a representing a sketch of a hand including the joint and a figure 72b representing a sketch of a foot are marked on the disposing surface. Further, the figures are marked such that the fingers of the figures 72a and 72b extend in the grating pitch direction of the first absorption type grating 31. Further, the figures may be marked at a substantially central portion of the disposing surface of the subject table 15 so as to be superimposed with each other (see FIG. 13A). However, when a plurality of figures is superimposed so that it is difficult to distinguish individual figures, for example, different colors for the figures may be used. Further, in order to avoid the plurality of figures from being superimposed, for example, figures 72a' and 73a' obtained by reducing the figures 72a and 72b may be marked at the edge of the disposing surface to be parallel to each other (see FIG. 13B).

When the figures are marked to be superimposed at the substantially central portion of the disposing surface of the subject table 15, the subject H is disposed to be superimposed with the figure corresponding to the type. Further, when reduced figures are marked at the edge of the disposing surface to be parallel to each other, the subject H is disposed along the figure corresponding to the type of the subject H, so that the edge of the region of interest of the subject H is disposed so be substantially perpendicular to the grating pitch direction of the first absorption type grating 31 and clearly visualized in the phase contrast image.

In the X-ray photographing apparatus 1, the subject table 15 in which the X-ray irradiating unit 11 and the photographing unit 12 are accommodated is supported by an arm member 61 of the stand 13 and the subject table 15 is attached to the arm member 61 such that the edge thereof along the grating pitch direction (the x direction) of the first absorption type grating 31 is disposed along the arm member 61 (see FIGS. 1 and 11). That is, the arm member 61 extends in a vertical direction (the z direction) to get out of a region in which the first absorption type grating 31 extends in the grating pitch direction. According to this configuration, the arm member 61 is not located in front of the subject table 15 in the grating pitch direction of the first absorption type grating 31. Therefore, specifically, when a relatively long subject, such as a leg or an arm, is disposed on the subject table 15, the subject is guided to be disposed in the relation with the grating pitch direction of the first absorption type grating 31 so that the subject may be appropriately disposed on the subject table 15 while avoiding the interference of the subject and the arm member 61.

As described above, according to the X-ray photographing apparatus 1, the subject H may be appropriately disposed in the relation with the grating pitch direction of the first absorption type grating 31 and a clear phase contrast image of the region of interest of the subject H may be obtained.

Further, most of the X-rays are not diffracted from the first absorption type grating 31 but geometrically projected onto the second absorption type grating 32 so that a high spatial coherency is not required for the irradiated X-ray and thus a general X-ray source which is used in a medical field may be used. Further, a distance $L_2$ from the first absorption type grating 31 to the second absorption type grating 32 may have an arbitrary value and the distance $L_2$ may be set to be smaller than a minimum Talbot interference distance of the Talbot interferometer so that a size (a thickness) of the photographing unit 12 may be reduced. Further, almost all wavelength components of the irradiated X-ray contribute to the projection image (G1 image) from the first absorption type grating 31 and a contrast of the moire stripe is improved so that a detecting sensitivity of the phase information of the subject H may be improved.

Even though it has been described that the second grating is superimposed with the projection image of the first grating to generate moire stripes so that both the first and second gratings are absorption type gratings, the present invention is not limited thereto. As described above, even when the moire stripe is generated by superimposing the Talbot interference image with the second grating, the present invention is useful. Therefore, the first grating is not limited to the absorption type grating, but may be a phase type grating.

Further, even though it is described that the phase shift distribution $\Phi$ which is represented as an image is stored or displayed as a phase contrast image, the phase shift distribution $\Phi$ is obtained by integrating a differential amount of the phase shift distribution $\Phi$ which is obtained from the refraction angle $\varphi$ and differential amounts of the refraction angle $\varphi$ and the phase shift distribution $\Phi$ are also related with the phase change of the X-ray by the subject. Therefore, the refraction angle $\varphi$ which is represented as an image and the differential amount of the phase shift which is represented as an image are also included in the phase contrast image.

Further, the phase contrast image generating process described above is performed on the moire stripes acquired by photographing (pre photographing) without a subject to obtain the phase contrast image. The phase contrast image reflects, for example, a phase irregularity (deviation of the initial phase) which is caused by the irregularity of the first and second absorption type gratings 31 and 32. When the phase contrast image in the pre photographing is subtracted from a phase contrast image acquired by photographing (main photographing) with a subject, a phase contrast image from which a phase irregularity of the photographing unit 12 is corrected may be obtained.

Further, even though it has been described that the photographing unit 12 is accommodated in the subject table 15, the photographing unit 12 and the subject table 15 may be separately configured. The refraction of the X-ray which is generated when transmitting the subject H is merely several μrad, and a phase change of a signal, which is obtained by modulating a periodic pattern (moire stripes) of an image generated by the refraction and analyzing the periodic pattern, is small. When such a small change is measured, a deviation of a relative position of the X-ray focus 18b, the first and second absorption type gratings 31 and 32, or the X-ray image detector 30, affects a detecting precision of the phase information of the subject H. When the photographing unit 12 is accommodated in the subject table 15 so that vibration which affects the detecting precision influences the photographing unit 12 through the photographing table 15, the photographing unit 12 and the subject table 15 are separately configured to reduce the influence thereof. However, it is desirable that the subject H and the first absorption type grating 31 are close to each other and thus, even when the photographing unit 12 and the subject table 15 are separately configured, it is desirable to dispose the subject and the first absorption type grating as close as possible.

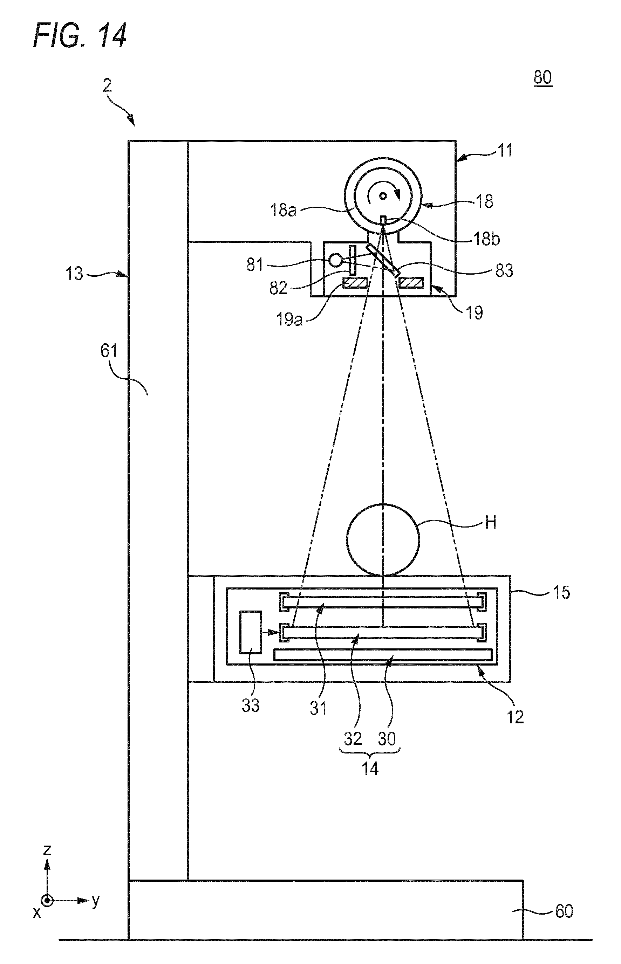

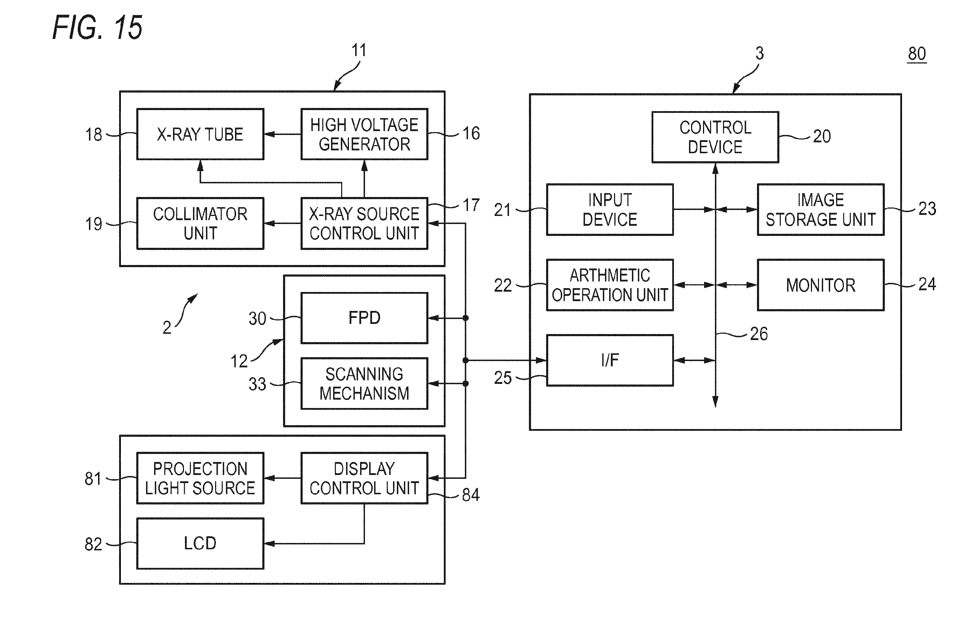

FIG. 14 illustrates a configuration of another example of a radiation photographing apparatus for describing an exemplary embodiment of the present invention and FIG. 15 is a control block diagram of the radiation photographing apparatus of FIG. 14. Further, like reference numerals denote like components of the X-ray photographing apparatus 1 and descriptions thereof will be omitted or simplified.

In the above-described X-ray photographing apparatus 1, an indicator which guides the arrangement of the subject H is marked on the disposing surface of the subject table 15 in advance. In contrast, in an X-ray photographing apparatus 80 illustrated in FIGS. 14 and 15, a type of the subject is input by an input device 21 of a console 3 and an indicator in accordance with the input type of subject is displayed on the disposing surface of the subject table 15 and the arrangement of the subject H on the disposing surface of the subject table 15 is guided by the displayed indicator.

An X-ray irradiating unit 11 includes a projection light source 81, a liquid crystal display (LCD) 82, and a mirror 83.

The mirror 83 transmits an X-ray which is radiated from an X-ray tube 18 and reflects light which is irradiated by the projection light source 81. In the illustrated example, the X-ray tube 18 and the projection light source 81 are disposed in an optically equivalent position. That is, even though the X-ray tube 18 and the projection light source 81 are disposed at different positions on a physical space, they are disposed at the same position when the returns thereof by the mirror 83 extend as straight lines.

The LCD 82 is a known projection type liquid crystal display device which is used for a projector and displays a sketch image of the subject in accordance with the type of subject input by the input device 21 of the console 3. A display control unit 84 obtains information on the type of subject which is input by the input device 21 and controls the LCD 82 to display the sketch image of the subject in accordance with the obtained type of subject and controls the projection light source 81 to be turned ON or OFF.

The projection light which is emitted from the projection light source 81 and transmits the LCD 82 to carry a display image of the LCD 82 is limited by a collimator unit 19 which is substantially the same irradiated field as an irradiated field of the X-ray radiated from the X-ray tube 18 and projected onto a disposing surface of the subject table 15.

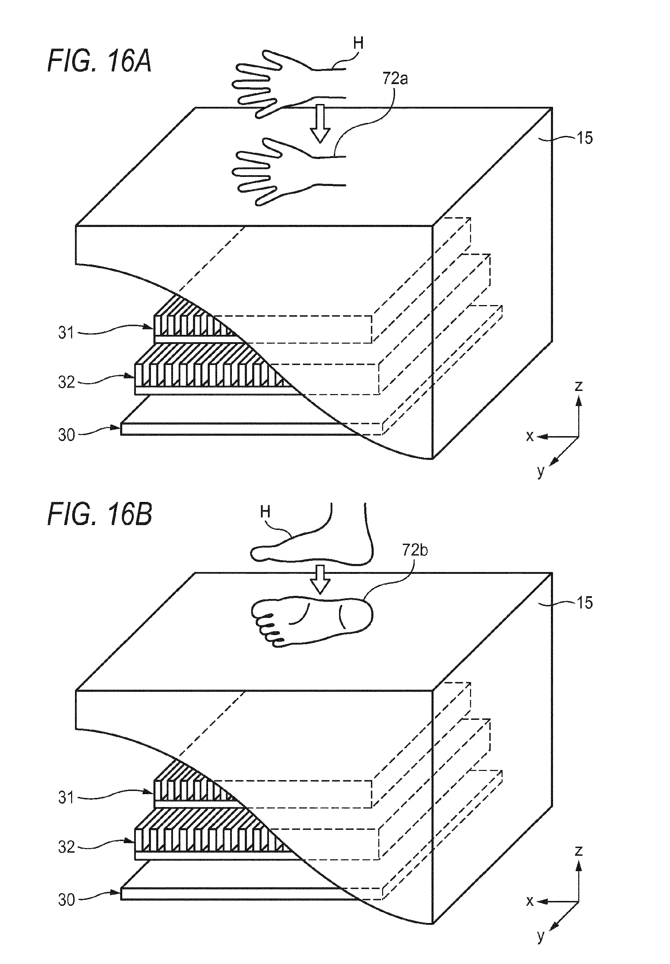

FIGS. 16A and 16B illustrate an example of an indicator which is displayed on the disposing surface of the subject table 15.

As a type of subject, in the example of interphalangeal joints of a hand and interphalangeal joints of a foot, a figure 72a (see FIG. 16A) representing a sketch of a hand including the joints and a figure 72b (see FIG. 16B) representing a sketch of a foot are selectively displayed at a substantially center of the disposing surface of the subject table 15 in accordance with the type of subject which is input by the console 3. Further, the figures are displayed such that the fingers of the figures 72a and 72b extend in the grating pitch direction of the first absorption type grating 31. The subject H is disposed to be superimposed with the figure which is displayed on the disposing surface so that an edge of a region of interest of the subject H is substantially perpendicular to a grating pitch direction of the first absorption type grating 31 and clearly visualized in the phase contrast image.

According to the above-described configuration, one figure in accordance with the type of subject H is displayed at the substantially center of the disposing surface of the subject table. Therefore, it is easy to distinguish the figure as compared with a case when a plurality of figures for every type of subject is marked to overlap each other on the disposing surface.

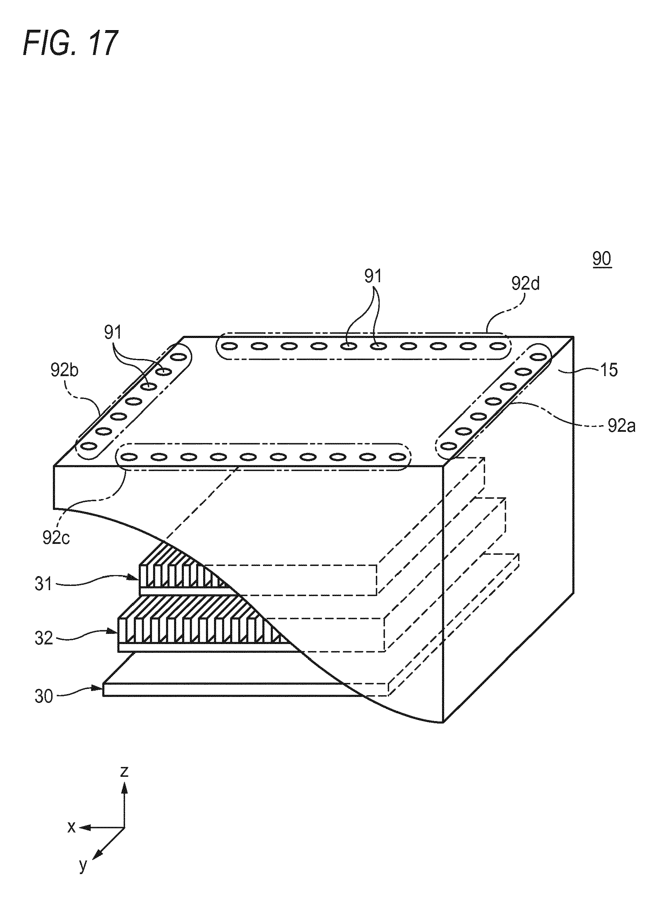

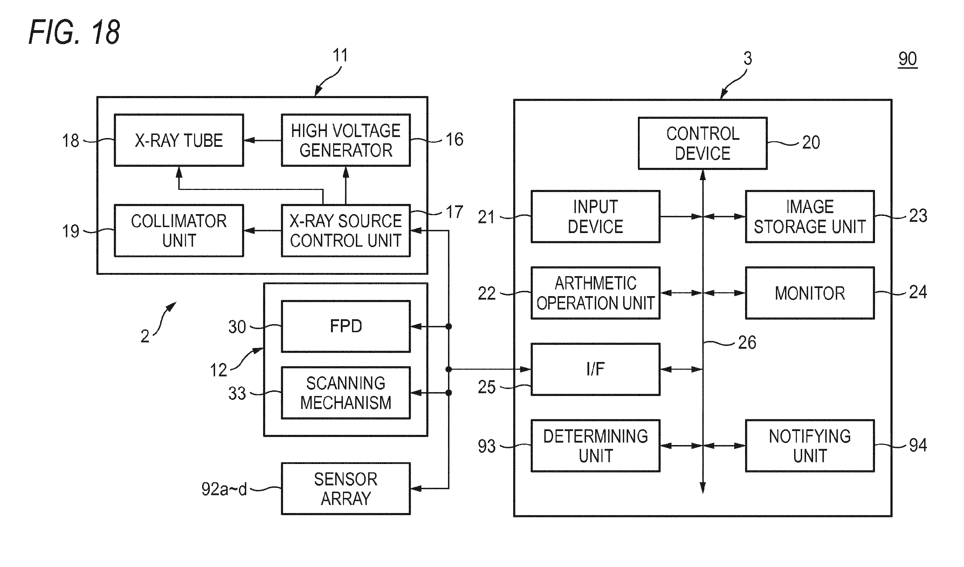

FIG. 17 illustrates a configuration of another example of a radiation photographing apparatus according to an exemplary embodiment of the present invention and FIG. 18 is a control block diagram of the radiation photographing apparatus of FIG. 17. Further, like reference numerals denote like components of the X-ray photographing apparatus 1 and descriptions thereof will be omitted or simplified.

In an X-ray photographing apparatus 90 illustrated in FIGS. 17 and 18, a detecting unit which detects a subject H is provided in a subject table 15 and the arrangement of the subject H on a disposing surface of the subject table 15 is guided based on a detection result of the subject H by the detecting unit. Other configurations are the same as those of the above-described X-ray photographing apparatus 1 but are not illustrated in the drawings.

At edges along sides of the subject table 15, sensor arrays 92a to 92d in which a plurality of sensors 91 as a detecting unit which detects the subject H is juxtaposed are provided. The subject H is superimposed on such sensor arrays 92 to detect the subject H. For example, a pressure sensor or a reflective photo sensor may be used as the sensor 91. In the present example, even though the sensor lines 92 are provided at the edges along the sides of the subject table 15, the sensor arrays may be provided at a pair of edges which intersect at least the grating pitch direction (the x direction) o the first absorption type grating 31.

The console 3 includes a determining unit 93 which acquires information detected in the sensor arrays 92a to 92d and determines whether the subject H is appropriately disposed on the disposing surface of the subject table 15 based on the acquired detection information, and a notifying unit 94 which notifies an operator of a determined result based on the determining result by the determining unit 93.

The determining unit 93 determines that the subject H is appropriately disposed when the subject H is detected in at least one of the pair of sensor arrays 92a and 92b which are provided at the edges which intersect the grating pitch direction (the x direction) of the first absorption type grating 31, among the sensor arrays 92a to 92d which are provided at the edges along the sides of the subject table 15 and determines that the subject H is not appropriately disposed when the subject H is detected in at least one of the pair of sensor arrays 92c and 92d which are provided at the edges which is parallel to the grating pitch direction (the x direction) of the first absorption type grating 31.

In the present example, the notifying unit 94 is configured to acquire information on the determining result in the determining unit 93 and display the obtained information on the determining result on a monitor 24 of the console 3.

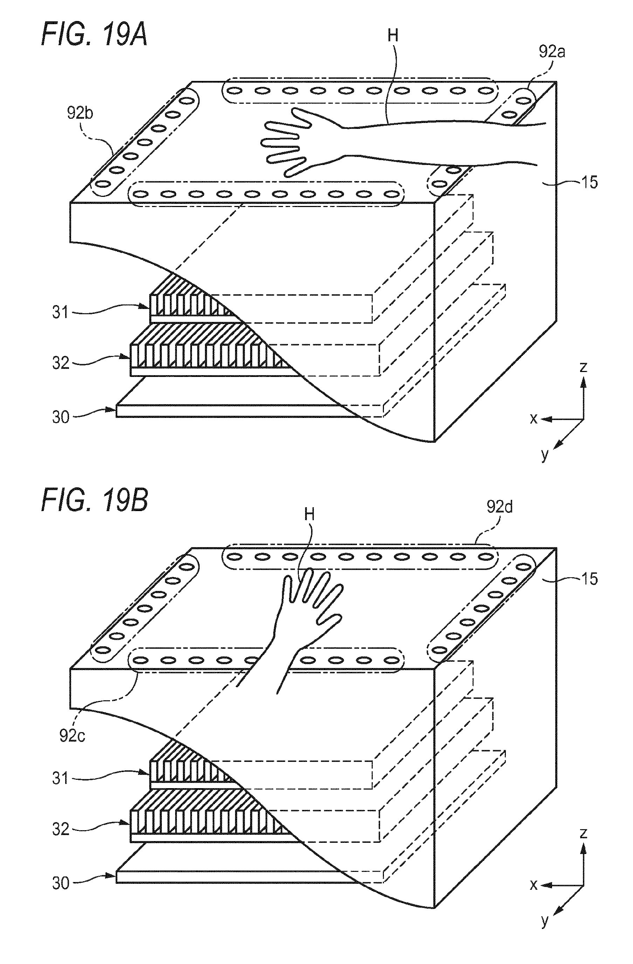

FIGS. 19A to 20B illustrate an example of the arrangement of the subject H on the disposing surface of the subject table 15.

FIGS. 19A and 19B illustrate a case in which the subject H is interphalangeal joints of a hand. When a pair of bones which form the interphalangeal joints are disposed substantially along the grating pitch direction (the x direction) of the first absorption type grating 31 (see FIG. 19A), an arm is superimposed with the sensor array 92a (or 92b) which is provided at the edge intersecting the grating pitch direction of the first absorption type grating 31. In this case, the determining unit 93 determines that the subject H is appropriately disposed and the determined result is displayed on the monitor 24 of the console 3 by the notifying unit 94.

When a pair of bones which form the interphalangeal joints are disposed to be substantially perpendicular to the grating pitch direction (the x direction) of the first absorption type grating 31 (see FIG. 19B), the arm is superimposed with the sensor array 92c (or 92d) which is provided at the edge which is parallel to the grating pitch direction of the first absorption type grating 31. In this case, the determining unit 93 determines that the subject H is not appropriately disposed and the determined result is displayed on the monitor 24 of the console 3 by the notifying unit 94. Therefore, the determined result which is displayed on the monitor 24 is referenced by the operator so that the arrangement of the subject H may be appropriately corrected.

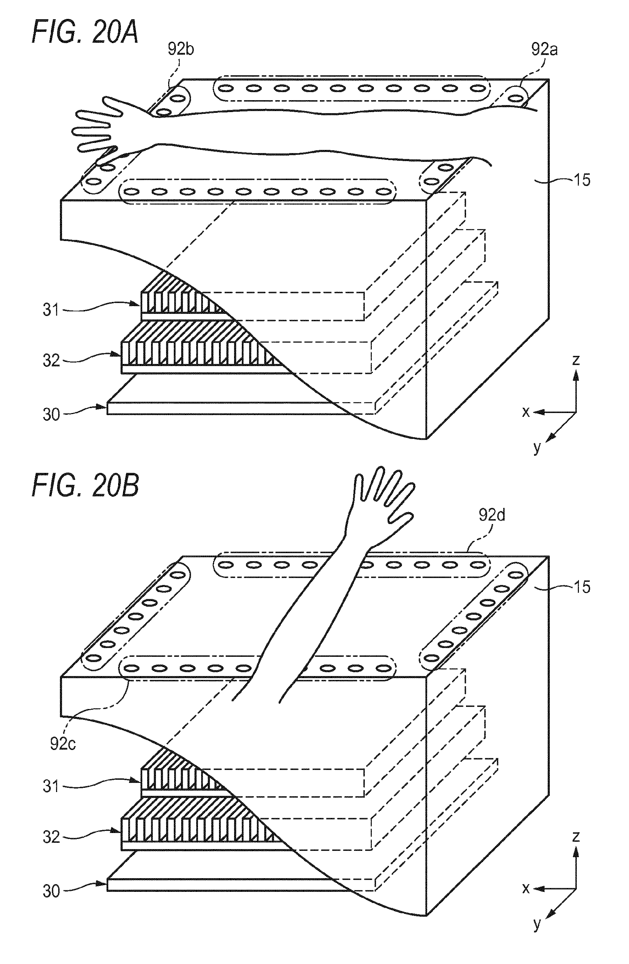

FIGS. 20A and 20B illustrate a case in which the subject is an elbow joint of the arm.

When an upper-arm bone and a radius which form the elbow joint are disposed approximately along the grating pitch direction (the x direction) of the first absorption type grating 31 (see FIG. 20A), the upper-arm and the lower-arm are superimposed with the sensor arrays 92a and 92b which are provided at the edges intersecting the grating pitch direction of the first absorption type grating 31. The determining unit 93 determines that the subject H is appropriately disposed and the determined result is displayed on the monitor 24 of the console 3 by the notifying unit 94.

When the upper-arm bone and the radius which form the elbow joint are disposed to be approximately perpendicular to the grating pitch direction (the x direction) of the first absorption type grating 31 (see FIG. 20B), the upper-arm and the lower-arm are superimposed with the sensor arrays 92c and 92d which are provided at the edges which are parallel to the grating pitch direction of the first absorption type grating 31. In this case, the determining unit 93 determines that the subject H is not appropriately disposed and the determined result is displayed on the monitor 24 of the console 3 by the notifying unit 94. Therefore, the operator refers to the determining result which is displayed on the monitor 24 to approximately correct the arrangement of the subject H.

By doing this, the joints are appropriately disposed and the edge of cartilage portion of the joint which is a region of interest is disposed to be approximately perpendicular to the grating pitch direction of the first absorption type grating 31 and clearly visualized in the phase contrast image.

Further, an example which notifies the operator of the determined result of the determining unit 93 is not limited to the example which displays the determined result on the monitor 24 of the console 3, and for example, the determined result may be notified by an alarm or voice.

Further, while the determining unit 93 determines that the subject H is not appropriately disposed, the X-ray photographing apparatus 90 may be interlocked not to perform the photographing operation.

Further, the determining unit 93 may designate a sensor array to detect the subject H in accordance with the type of the subject which is input by the input device 21 of the console 3. For example, when the subject is the interphalangeal joint of the hand (see FIGS. 19A and 19B), the interphalangeal joint of the hand is input by the input device 21 as the type of the subject. The determining unit 93 designates the sensor array 92a (or 92b) which is provided at the edge intersecting the grating pitch direction of the first absorption type grating 31 based on the input information and determines that the subject is appropriately disposed when the subject is detected by the designated sensor array 92a. Further, when the subject is the elbow joint (see FIGS. 20A and 20B), the elbow joint is input by the input device 21 as the type of subject. The determining unit 93 designates the sensor arrays 92a and 92b based on the input information and determines that the subject is appropriately disposed when the subject is detected by the designated sensor arrays 92a and 92b.

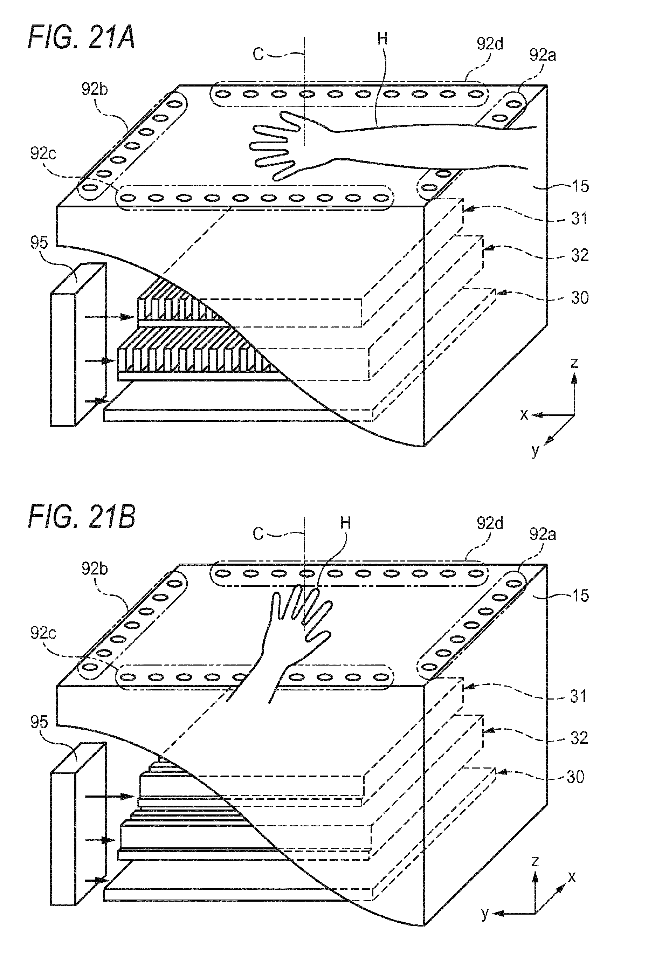

FIGS. 21A and 21B illustrate a configuration of a modification embodiment of the above-described X-ray photographing apparatus 90.

In the example illustrated in FIGS. 21A and 21B, the rotating mechanism 95 which rotates the first and second absorption type gratings 31 and 32 and the X-ray image detector 30 around the optical axis C while maintaining the relative positional relationship therebetween is provided.

In a case in which the subject H is the interphalangeal joints of the hand, when the subject is detected by the sensor array 92c (or 92d) which is provided at the edge which is parallel to the grating pitch direction of the first absorption type grating 31 (see FIG. 21A), the determining unit 93 determines that the subject is not appropriately disposed and the control device 20 of the console 3 drives the rotating mechanism 95 to rotate the first and second absorption type gratings 31 and 32 and the X-ray image detector 30 by approximately 90 degrees (see FIG. 21B).

In accordance with the rotation of the first absorption type grating 31, a pair of bones which form the interphalangeal joint are disposed approximately along the grating pitch direction (the x direction) of the first absorption type grating 31. Further, a pair of sensor arrays which are disposed at the edge intersecting the grating pitch direction (the x direction) of the first absorption type grating 31 are sensor arrays 92c and 92d and when the subject H is detected by at least one sensor array of the sensor arrays 92c and 92d, the control device 20 changes a setting to determine that the subject H is appropriately disposed so that the determining unit 93 determines that the subject is appropriately disposed.

With this configuration, an appropriate arrangement of the subject in relation to the grating pitch direction of the first absorption type grating 31 is automatically achieved.

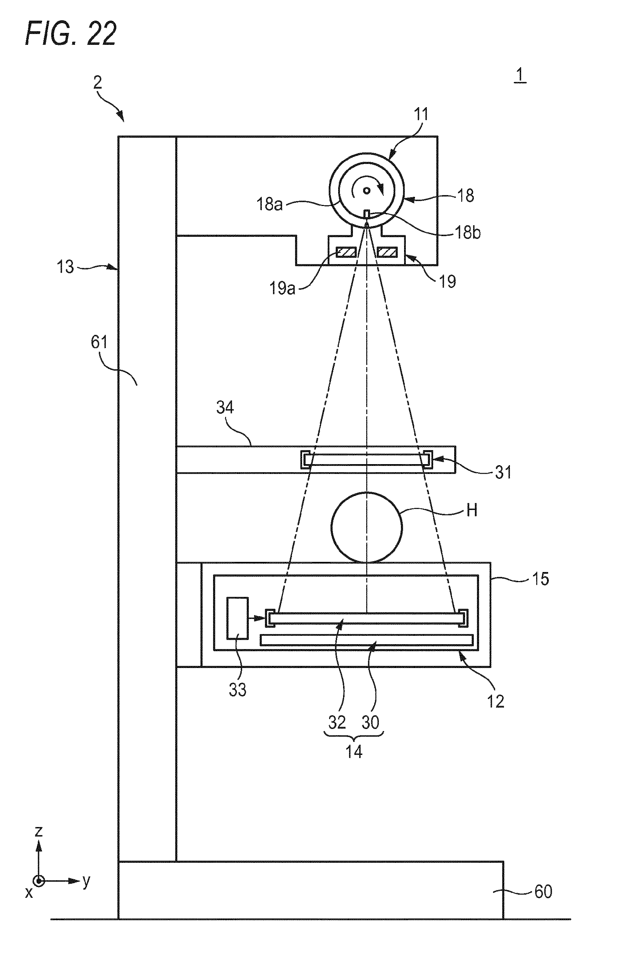

FIG. 22 illustrates a configuration of another example of a radiation photographing apparatus for describing an exemplary embodiment of the present invention.

An X-ray photographing apparatus illustrated in FIG. 22 is different from the X-ray photographing apparatus 1 illustrated in FIG. 1 in that a first absorption type grating 31 is disposed between an X-ray irradiating unit 11 and a subject table 15. The first absorption type grating 31 is accommodated in a holding member 34 which is attached to an arm member 61.

As a guide unit which guides the arrangement of a subject H on a disposing surface of the subject table 15, various guide units which have been described in the X-ray photographing apparatuses 1, 80, and 90 may be used. When the guide unit is configured by the indicator in the X-ray photographing apparatuses 1 and 80, an indicator may be marked or displayed on the disposing surface of the subject table 15 similarly to the X-ray photographing apparatuses 1 and 80. However, the indicator may be marked or displayed on a surface of the holding member 34. When the indicator is displayed on the disposing surface of the subject table 15 using projection light like the X-ray photographing apparatus 80, the holding member 34 is formed of a material which transmits the projection light not to shield the projection light by the holding member 34 and the first absorption type grating 31 and the first absorption type grating 31 may be configured to be moved in the holding member 34 so as to be out of the irradiated field before performing an image capturing process.

In the above configuration, the subject H is disposed between the first absorption type grating 31 and the second absorption type grating 32 but the G1 image of the first absorption type grating 31 which is formed in the position of the second absorption type grating 32 is modulated by the subject H. Similarly to the above-described X-ray photographing system 1, an intensity of the G1 image is modulated by the superimposition with the second absorption type grating 32 and the G1 image of which the intensity is modulated is captured by the X-ray image detector 30. Therefore, also in the X-ray photographing system, a phase contrast image of the subject H may be obtained in accordance with the above-described principle.

In the X-ray photographing apparatus, an X-ray of which the dose is reduced by almost half due to the shielding by the first absorption type grating 31 is irradiated onto the subject H so that an exposed dose of the subject H may be reduced by approximately half of the above-described X-ray photographing system 1.

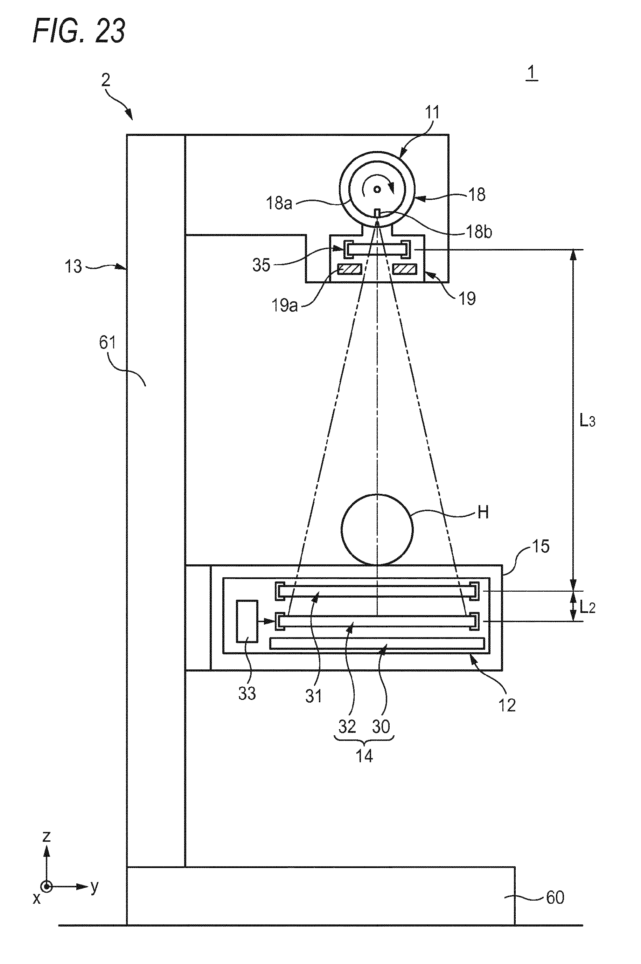

FIG. 23 illustrates a configuration of another example of a radiation photographing apparatus according to an exemplary embodiment of the present invention.

The X-ray photographing apparatus illustrated in FIG. 23 is different from the above-described X-ray photographing apparatus 1 in that a multi slit 35 is disposed in the X-ray irradiating unit 11.

In the above-described X-ray photographing apparatus 1, when a distance from the X-ray irradiating unit 11 to the X-ray image detector 30 is set to be a distance (1 m to 2 m) which is set in an X-ray room of a general hospital, due to blurring of a G1 image by a focus size (generally, approximately 0.1 mm to 1 mm) of the X-ray focus 18b, an image quality of the phase contrast image may be degraded. Therefore, even though it is considered that a pinhole is provided directly after the X-ray focus 18b to effectively reduce a focus size, if an opening area of the pin hole is reduced in order to reduce the effective focus size, the intensity of the X-ray is lowered. In this example, in order to solve the above problem, the multi slit 35 is disposed directly after the X-ray focus 18b.

The multi slit 35 is an absorption type grating (a third absorption type grating) with the same configuration as the first and second absorption type gratings 31 and 32 and a plurality of X-ray shielding units which extends in one direction is periodically arranged in the same direction (x direction) as the X-ray shielding units 31b and 32b of the first and second absorption type gratings 31 and 32. The multi slit 35 partially shields the radiation which is radiated from the X-ray focus 18b so as to form a plurality of microfocus light sources (dispersed light sources) which is arranged in an x direction with a predetermined pitch.

When a distance from the multi slit 35 to the first absorption type grating 31 is $L_3$, a grating pitch $p_3$ of the multi slit 35 needs to be set so as to satisfy the following Equation 20.

[Equation 20]

$$p_3 = \frac{L_3}{L_2} p_2 \quad \cdots (20)$$

Equation 20 is a geometrical condition in which a projection image (a G1 image) of the X-ray which is emitted from each point light source which is dispersed by the multi slit 35 by the first absorption type grating 31 matches (is superimposed) with the position of the second absorption type grating 32.

Further, the position of the multi slit 35 substantially becomes the X-ray focal position so that a grating pitch $p_2$ of the second absorption type grating 32 is determined so as to satisfy the relationship of the following Equation 21.

[Equation 21]

$$p_2 = \frac{L_3 + L_2}{L_3} p_1 \quad \cdots (21)$$

As a guide unit which guides the arrangement of a subject H on a disposing surface of the subject table 15, various guide units which have been described in the X-ray photographing apparatuses 1, 80, and 90 may be used. When an indicator serving as a guide is displayed on the disposing surface of the subject table 15 using the projection light, like the X-ray photographing apparatus 80, the multi slit 35 is disposed between the mirror 83 and the X-ray tube 18 so as not to shield the projection light by the multi slit 35. Further, similarly to the modification embodiment of the X-ray photographing apparatus 90, when the first and second absorption type gratings 31 and 32 and the X-ray image detector 30 rotate in accordance with the arrangement of the subject, the multi slit 35 also rotates together with the first and second absorption type gratings 31 and 32 and the X-ray image detector 30.

In the above configuration, the G1 image based on the plurality of point light sources which is formed by the multi slit 35 is superimposed so that an image quality of the phase contrast image may be improved without lowering the intensity of the X-ray.

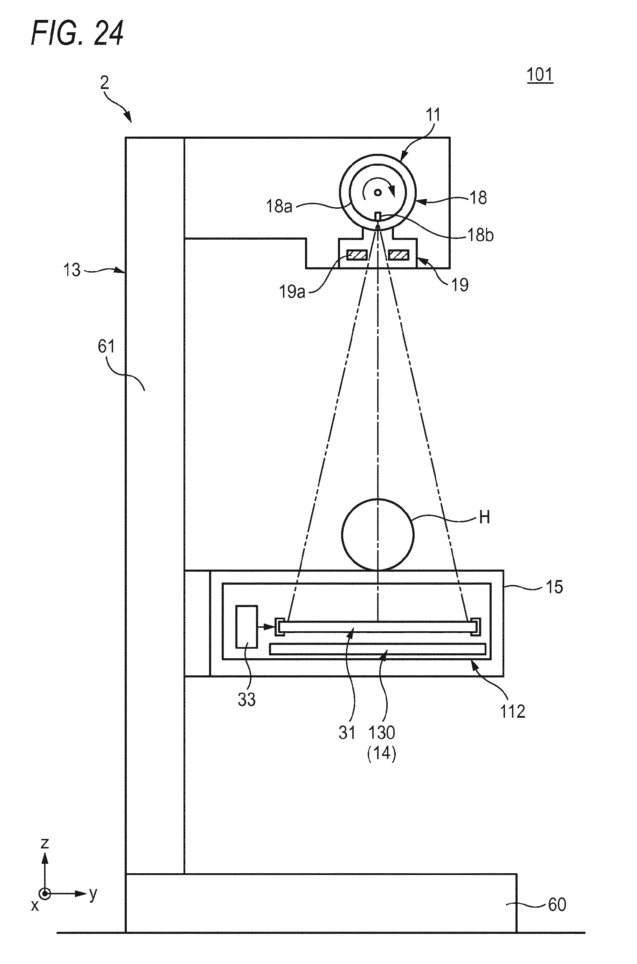

FIG. 24 illustrates a configuration of another example of a radiation photographing apparatus for describing an exemplary embodiment of the present invention.

An X-ray photographing apparatus 101 is mainly divided into a main body 102 of an X-ray photographing apparatus and a console 3. In the main body 102 of an X-ray photographing apparatus, a photographing unit 112 which is accommodated in an image capturing table 15 includes a first absorption type grating 31 and an X-ray image detector 130 which detects a G1 image formed by an X-ray which passes through the first absorption type grating 31.

In the X-ray image detector 130, a plurality of pixels is arranged with an arrangement pitch to resolve the periodic intensity distribution of the G1 image which is formed on a receiving surface of the X-ray image detector 130. An image which is obtained by capturing the G1 image using the X-ray image detector 130 includes a periodic pattern corresponding to the periodic intensity distribution of the G1 image and the periodic pattern is analyzed to generate a phase contrast image of the subject H. The periodic pattern included in the image is analyzed using the above-described stripe scanning method, Fourier transformation, and inverse Fourier transformation. When the pattern is analyzed using the stripe scanning method, the image may be captured while translationally moving the first absorption type grating 31 in a grating pitch direction (the x direction) of the grating with respect to the X-ray image detector 130 stepwise and relatively changing a phase of the periodic arrangement of the pixel with respect to the periodic intensity distribution of the G1 image.

In the X-ray photographing apparatus 101, as a guide unit which guides the arrangement of a subject H on a disposing surface of the subject table 15, various guide units which have been described in the X-ray photographing apparatuses 1, 80, and 90 may be used

As described above, according to the X-ray photographing apparatus 101, the periodic intensity distribution of the G1 image is detected using a detector with a pixel pitch which is smaller than a period of the periodic intensity distribution of the G1 image, the periodic intensity distribution is analyzed to obtain phase information, and the pixel pitch is small so that spatial resolving power is excellent. Further, the X-ray does not pass through the second absorption type grating 32 so that precision of the phase information is improved.

Further, the arrangement pitch of the pixel in the X-ray image detector 130 is an arrangement pitch which generates moire in the relationship with the period of the periodic intensity distribution o the G1 image which is formed on the image receiving surface of the X-ray image detector 130 and the moire which is included in the image which is obtained by the X-ray image detector 130 is analyzed so as to generate the phase contrast image of the subject H. Generally, as the pixel in the X-ray image detector becomes smaller, an S/N tends to be lowered. Therefore, it does not need to reduce the arrangement pitch of the pixel in the X-ray image detector so as to detect a minute periodic intensity distribution of the G1 image but an S/N may be secured to increase the precision of the phase information. The moire which is included in the image may be analyzed, for example, using the above-described stripe scanning method, Fourier transformation, and inverse Fourier transformation.

Further, also in the X-ray photographing apparatus 101, the first absorption type grating 31 may be disposed between the X-ray irradiating unit 11 and the subject table 15.

Further, also in the X-ray photographing apparatus 101, the above-described multi slit 35 (see FIG. 23) may be provided in the X-ray irradiating unit 11. In this case, when a distance from the multi slit to the first absorption type grating 31 is $L_3$, and a distance from the first absorption type grating 31 to the X-ray image detector 130 is $L_4$, the grating pitch $p_3$ of the multi slit is set to satisfy the following Equation 22.

[Equation 22]

$$p_3 = \frac{L_3 + L_4}{L_4} p_1 \quad \cdots (22)$$

Equation 22 is a geometrical condition in which a projection image (a G1 image) of the X-ray which is emitted from each point light source which is dispersed by the multi slit 35 by the first absorption type grating 31 matches (is superimposed) with the position of the X-ray image detector 30.

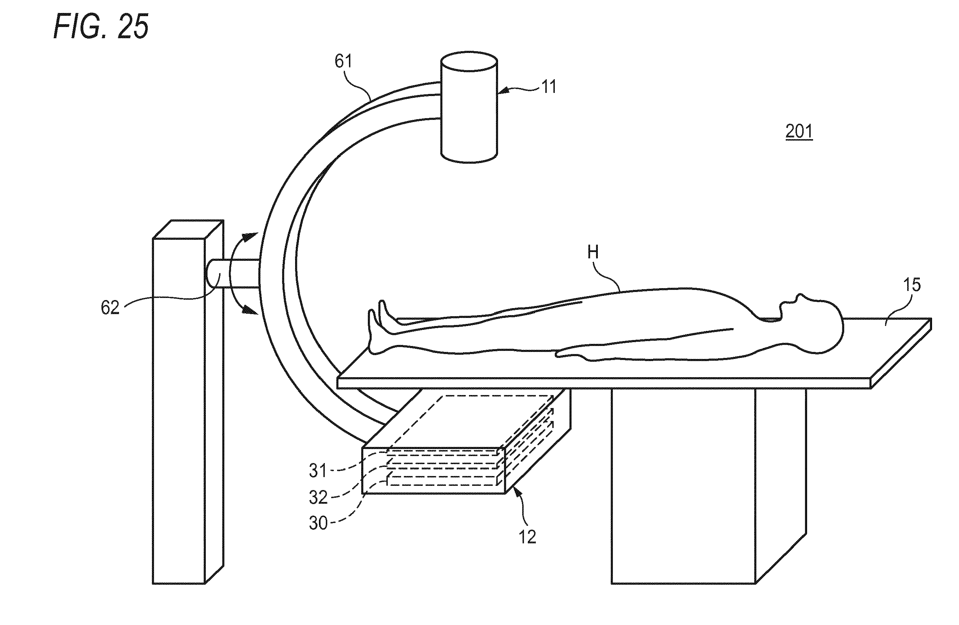

FIG. 25 illustrates a configuration of another example of a radiation photographing apparatus according to an exemplary embodiment of the present invention.

An X-ray photographing apparatus 201 illustrated in FIG. 25 is different from the above-described X-ray photographing apparatus 1 in that the photographing unit 12 in which the first absorption type grating 31, the second absorption type grating 32, and the X-ray image detector 30 are accommodated and the subject table 15 are separately provided and an X-ray irradiating unit 11 and the photographing unit 12 are supported by the arm member 61 and the arm member 61 pivots around a pivot shaft 62.

According to the X-ray photographing apparatus 201, the subject H may be photographed from various directions as the arm member 61 pivots.

As a guide unit which guides the arrangement of a subject H on the subject table 15, various guide units which have been described in the X-ray photographing apparatuses 1, 80, and 90 may be used. Further, the subject table 15 may not be essential for some subjects H. When the subject table 15 is not used, an indicator 70, 71, or 72 or a sensor 91 which guides the arrangement of the subject H may be provided or displayed on a case of the photographing unit 12.

Also in the X-ray photographing apparatus 201, the first absorption type grating 31 may be disposed between the X-ray irradiating unit 11 and the subject H (see FIG. 22) and in this case, a supporting unit which supports the first absorption type grating 31 may be provided in the arm member 61.

Further, also in the X-ray photographing apparatus 201, the above-described multi slit 35 (see FIG. 23) may be provided in the X-ray irradiating unit 11.

Further, also in the X-ray photographing apparatus 201, similarly to the above-described X-ray photographing apparatus 101, a periodic pattern corresponding to the periodic intensity distribution of the G1 image which is included in an image acquired by the X-ray image detector is analyzed using the X-ray image detector which may resolve the periodic intensity distribution of the G1 image to generate a phase contrast image of the subject H.

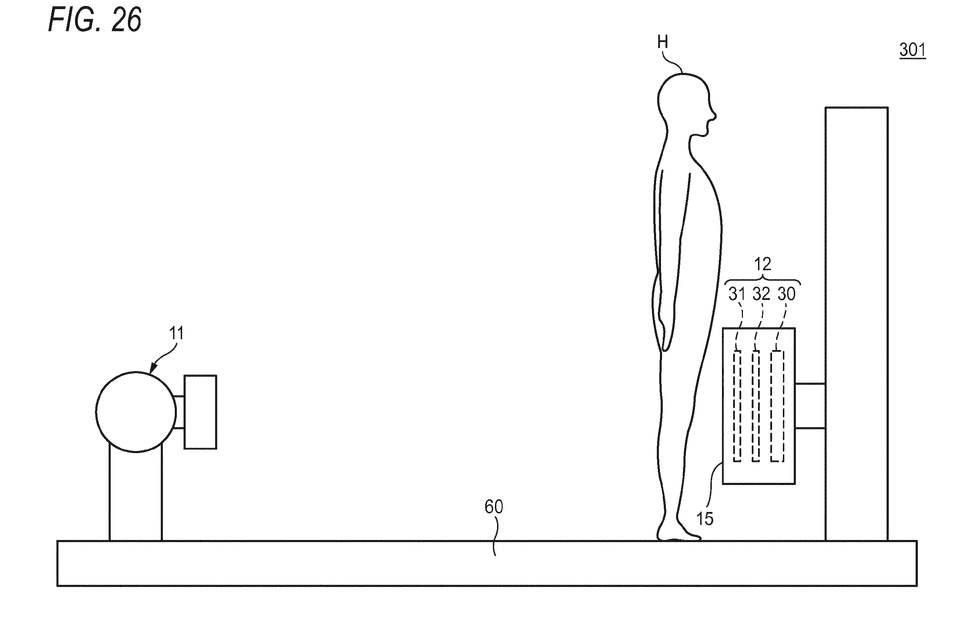

FIG. 26 illustrates a configuration of another example of a radiation photographing apparatus for describing an exemplary embodiment of the present invention.

An X-ray photographing apparatus 301 illustrated in FIG. 26 is different from the above-described X-ray photographing apparatus 1 which irradiates the X-ray approximately in a lower vertical direction and basically photographs the subject H in a lying posture or a seated posture in that the subject H is photographed in a standing posture and the X-ray is irradiated approximately in a horizontal direction.

In the X-ray photographing apparatus 301, an X-ray irradiating unit 11 and a photographing unit 12 which includes a first absorption type grating 31, a second absorption type grating 32, and an X-ray image detector 30 are provided in a base 60 approximately in a horizontal direction to be parallel to each other.

As a guide unit which guides the arrangement of a subject H on the subject table 15, various guide units which have been described in the X-ray photographing apparatuses 1, 80, and 90 may be used.

Further, also in the X-ray photographing apparatus 301, the first absorption type grating 31 may be disposed between the X-ray irradiating unit 11 and the subject H (see FIG. 22).

Further, also in the X-ray photographing apparatus 301, a multi slit 35 (see FIG. 23) may be provided in the X-ray irradiating unit 11.

Further, also in the X-ray photographing apparatus 301, similarly to the above-described X-ray photographing apparatus 101, a periodic pattern corresponding to the periodic intensity distribution of the G1 image which is included in an image obtained by the X-ray image detector is analyzed using the X-ray image detector which may resolve the periodic intensity distribution of the G1 image to generate a phase contrast image of the subject H.

In the above description, it is described that a general X-ray is used as radiation, but the present invention is not limited to the X-ray and may use radiation such as a $\alpha$ ray or a $\gamma$ ray other than the X-ray. Further, as for X-ray photographing apparatuses other than the above-described X-ray photographing apparatus 1, similarly to the X-ray photographing apparatus 1, the first grating is not limited to the absorption type grating but a phase type grating may be used.

As described above, in this specification, radiation photographing apparatuses of (1) to (22) are disclosed.

(1) A radiation photographing apparatus, including: a radiation irradiating unit; a first grating which has a periodic structure which is formed by arranging a plurality of linear bodies and forms a radiation image including a periodic intensity distribution by passing radiation; a detecting unit which acquires radiation image data by detecting the radiation image which is irradiated from the radiation irradiating unit to transmit the first grating and a subject and subjected to modulation for the periodic intensity distribution; and a guide unit which guides an arrangement of the subject in relation to an arrangement direction of a group of the linear bodies of the first grating.

(2) The radiation photographing apparatus of (1), further including: a subject table, on which the subject is disposed, between the radiation irradiating unit and the detecting unit, wherein the guide unit is an indicator marked on the subject table.

(3) The radiation photographing apparatus of (2), wherein the subject table is disposed between the radiation irradiating unit and the first grating or between the first grating and the detecting unit.

(4) The radiation photographing apparatus of (2) or (3), wherein the indicator indicates the arrangement direction of the group of the linear bodies of the first grating or an extending direction of the linear bodies of the first grating.

(5) The radiation photographing apparatus of (2) or (3), wherein a plurality of indicators is provided for each type of subject and indicates a sketch of the corresponding type of subject.

(6) The radiation photographing apparatus of (1), further including: a subject table, on which the subject is disposed, between the radiation irradiating unit and the detecting unit, wherein the guide unit has a display unit which displays an indicator on the subject table.

(7) The radiation photographing apparatus of (6), wherein the subject table is disposed between the radiation irradiating unit and the first grating or between the first grating and the detecting unit.

(8) The radiation photographing apparatus of (6) or (7), wherein a plurality of indicators is provided for every type of subject and indicates a sketch of the corresponding type of subject.

(9) The radiation photographing apparatus of (8), an input unit which inputs the type of subject, wherein the display unit displays the indicator in accordance with the type of subject which is input by the input unit.

(10) The radiation photographing apparatus of (1), further including: a subject table, on which the subject is disposed, between the radiation irradiating unit and the detecting unit, wherein the guide unit includes a subject detecting unit which detects an arrangement of the subject on the subject table and a determining unit which determines whether the subject is appropriately disposed based on a detecting result of the subject detecting unit.

(11) The radiation photographing apparatus of (10), wherein the subject table is disposed between the radiation irradiating unit and the first grating or between the first grating and the detecting unit.

(12) The radiation photographing apparatus of (10) or (11), wherein the subject detecting unit includes a plurality of sensors which is provided along edges of the subject table, when the subject is detected by a sensor which is provided at an edge intersecting the arrangement direction of the group of the linear bodies of the first grating, the determining unit determines that the subject is appropriately disposed, and when the subject is detected by a sensor which is provided at an edge along the arrangement direction of the group of the linear bodies of the first grating, the determining unit determines that the subject is inappropriately disposed.

(13) The radiation photographing apparatus of any one of (10) to (12), further including: a notifying unit which notifies that the subject is inappropriately disposed when the determining unit determines that the subject is inappropriately disposed.

(14) The radiation photographing apparatus of any one of (10) to (12), further including: a driving unit which rotates the first grating and the detecting unit around an optical axis of the radiation which passes through the first grating when the determining unit determines that the subject is inappropriately disposed.

(15) The radiation photographing apparatus of any one of (1) to (14), further including: an arm member which extends in an arrangement direction of the radiation irradiating unit, the first grating, and the detecting unit, and supports the radiation irradiating unit, the first grating, and the detecting unit.

(16) The radiation photographing apparatus of (15), wherein the arm member is disposed to be out of an extending region where the first grating extends in the arrangement direction of the linear bodies.

(17) The radiation photographing apparatus of any one of (1) to (16), wherein the detecting unit further includes a second grating which is superimposed with the radiation image and the radiation image detector detects the radiation image with which the second grating is superimposed.

(18) The radiation photographing apparatus of any one of (1) to (16), wherein the radiation image detector has a resolution which resolves the periodic intensity distribution of the radiation image and detects the radiation image.

(19) The radiation photographing apparatus of any one of (1) to (16), wherein the radiation image detector has a resolution which generates a moire in relation to a period of the periodic intensity distribution of the radiation image and detects the radiation image.

(20) The radiation photographing apparatus of any one of (1) to (19), further including: an arithmetic operation processing unit which generates a phase contrast image of the subject based on at least one piece of radiation image data which is obtained by the detecting unit of the radiation photographing apparatus.

(21) A radiation photographing apparatus, including: a radiation irradiating unit; a first grating which has a periodic structure which is formed by arranging a plurality of linear bodies and forms a radiation image including a periodic intensity distribution by passing radiation; a detecting unit which includes a radiation image detector and acquires radiation image data by detecting the radiation image which is subjected to modulation for the periodic intensity distribution by the subject disposed between the radiation irradiating unit and the detecting unit; and a guide unit which guides an arrangement of the subject in relation to an arrangement direction of a group of the linear bodies of the first grating.

(22) The radiation photographing apparatus of (21), wherein the subject is disposed between the radiation irradiating unit and the first grating or between the first grating and the detecting unit.

Industrial Applicability

The present invention provides a radiation photographing apparatus which allows radiation to be incident onto a subject from an appropriate direction and obtains a clear phase contrast image.

Although the present invention has been described in detail with reference to specific embodiments thereof, it is obvious to those skilled in the art that various changes or modifications may be made without departing from the spirit and scope of the present invention.

This application is based on Japanese Patent Application No. 2011-266164 filed on December 5, 2011 and Japanese Patent Application No. 2012-236818 filed on October 26, 2012, the entire contents of which are incorporated herein by reference.

Reference Signs List

1:      X-ray photographing apparatus
2:      Main body of X-ray photographing apparatus
3:      Console
11:     X-ray irradiating unit
12:     Photographing unit

14:     Detecting unit
15:     Subject table
18:     X-ray tube
30:     X-ray image detector
31:     First absorption type grating (first grating)
31a:    Substrate
31b:    X-ray shielding unit (linear body)
32:     Second absorption type grating (second grating)

**Claims**

1.  A radiation photographing apparatus, comprising:

    a radiation irradiating unit;
    a first grating which has a periodic structure which is formed by arranging a plurality of linear bodies and forms a radiation image including a periodic intensity distribution by passing radiation;
    a detecting unit which acquires radiation image data by detecting the radiation image which is irradiated from the radiation irradiating unit to transmit the first grating and a subject and subjected to modulation for the periodic intensity distribution; and
    a guide unit which guides an arrangement of the subject in relation to an arrangement direction of a group of the linear bodies of the first grating.

2.  The radiation photographing apparatus of claim 1, further comprising:

    a subject table, on which the subject is disposed, between the radiation irradiating unit and the detecting unit, wherein the guide unit is an indicator marked on the subject table.

3.  The radiation photographing apparatus of claim 2, wherein the subject table is disposed between the radiation irradiating unit and the first grating or between the first grating and the detecting unit.

4.  The radiation photographing apparatus of claim 2 or 3, wherein the indicator indicates the arrangement direction of the group of the linear bodies of the first grating or an extending direction of the linear bodies of the first grating.

5.  The radiation photographing apparatus of claim 2 or 3, wherein a plurality of indicators is provided for each type of subject and indicates a sketch of the corresponding type of subject.

6.  The radiation photographing apparatus of claim 1, further comprising:

    a subject table, on which the subject is disposed, between the radiation irradiating unit and the detecting unit, wherein the guide unit has a display unit which displays an indicator on the subject table.

7.  The radiation photographing apparatus of claim 6, wherein the subject table is disposed between the radiation irradiating unit and the first grating or between the first grating and the detecting unit.

8.  The radiation photographing apparatus of claim 6 or 7, wherein a plurality of indicators is provided for every type of subject and indicates a sketch of the corresponding type of subject.

9.  The radiation photographing apparatus of claim 8, further comprising:

    an input unit which inputs the type of subject,
    wherein the display unit displays the indicator in accordance with the type of subject which is input by the input unit.

10. The radiation photographing apparatus of claim 1, further comprising:

    a subject table, on which the subject is disposed, between the radiation irradiating unit and the detecting unit, wherein the guide unit includes a subject detecting unit which detects an arrangement of the subject on the subject table and a determining unit which determines whether the subject is appropriately disposed based on a detecting result of the subject detecting unit.

11. The radiation photographing apparatus of claim 10, wherein the subject table is disposed between the radiation irradiating unit and the first grating or between the first grating and the detecting unit.

12. The radiation photographing apparatus of claim 10 or 11, wherein the subject detecting unit includes a plurality of sensors which is provided along edges of the subject table,
when the subject is detected by a sensor which is provided at an edge intersecting the arrangement direction of the group of the linear bodies of the first grating, the determining unit determines that the subject is appropriately disposed, and
when the subject is detected by a sensor which is provided at an edge along the arrangement direction of the group of the linear bodies of the first grating, the determining unit determines that the subject is inappropriately disposed.

13. The radiation photographing apparatus of any one of claims 10 to 12, further comprising:

a notifying unit which notifies that the subject is inappropriately disposed when the determining unit determines that the subject is inappropriately disposed.

14. The radiation photographing apparatus of any one of claims 10 to 12, further comprising:

a driving unit which rotates the first grating and the detecting unit around an optical axis of the radiation which passes through the first grating when the determining unit determines that the subject is inappropriately disposed.

15. The radiation photographing apparatus of any one of claims 1 to 14, further comprising:

an arm member which extends in an arrangement direction of the radiation irradiating unit, the first grating, and the detecting unit, and supports the radiation irradiating unit, the first grating, and the detecting unit.

16. The radiation photographing apparatus of claim 15, wherein the arm member is disposed to be out of an extending region where the first grating extends in the arrangement direction of the linear bodies.

17. The radiation photographing apparatus of any one of claims 1 to 16, wherein the detecting unit further includes a second grating which is superimposed with the radiation image and the radiation image detector detects the radiation image with which the second grating is superimposed.

18. The radiation photographing apparatus of any one of claims 1 to 16, wherein the radiation image detector has a resolution which resolves the periodic intensity distribution of the radiation image and detects the radiation image.

19. The radiation photographing apparatus of any one of claims 1 to 16, wherein the radiation image detector has a resolution which generates a moire in relation to a period of the periodic intensity distribution of the radiation image and detects the radiation image.

20. The radiation photographing apparatus of any one of claims 1 to 19, further comprising:

an arithmetic operation processing unit which generates a phase contrast image of the subject based on at least one piece of radiation image data which is obtained by the detecting unit of the radiation photographing apparatus.

*FIG. 1*

# FIG. 2

1

3 → CONTROL DEVICE — 20

IMAGE STORAGE UNIT — 23

MONITOR — 24

26

INPUT DEVICE — 21

ARITHMETIC OPERATION UNIT — 22

I/F — 25

11

HIGH VOLTAGE GENERATOR — 16

X-RAY SOURCE CONTROL UNIT — 17

X-RAY TUBE — 18

COLLIMATOR UNIT — 19

FPD — 30

SCANNING MECHANISM — 33

12

2

FIG. 3

*FIG. 4*

FIG. 5

FIG. 6

*FIG. 7*

SHADE
PORTION
LIGHT
PORTION
$p_1'$

G1 IMAGE

32

32b

k=0

32

k=1

k=2

k=M/2

32

k=M-1

32b

SCANNING
DIRECTION

*FIG. 8*

WITH
SUBJECT

WITHOUT
SUBJECT

$I_k(x)$

POSITION (k)

0          M/2          M-1

$\psi$ : (PHASE DEVIATION AMOUNT)

## FIG. 9

FIG. 10

*FIG. 11*

FIG. 12

*FIG. 13A*

*FIG. 13B*

## FIG. 14

FIG. 15

EP 2 789 296 A1

FIG. 16A

FIG. 16B

## FIG. 17

## FIG. 18

EP 2 789 296 A1

FIG. 19A

FIG. 19B

FIG. 20A

FIG. 20B

*FIG. 21A*

*FIG. 21B*

FIG. 22

FIG. 23

FIG. 24

FIG. 25

FIG. 26

EP 2 789 296 A1

## INTERNATIONAL SEARCH REPORT

| International application No. |
| --- |
| PCT/JP2012/079005 |

**A. CLASSIFICATION OF SUBJECT MATTER**
*A61B6/00*(2006.01)i, *G01T7/00*(2006.01)i

According to International Patent Classification (IPC) or to both national classification and IPC

**B. FIELDS SEARCHED**

Minimum documentation searched (classification system followed by classification symbols)
A61B6/00, G01T7/00

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | WO 2011/011014 A1 (The United States of America, as Represented by the Secretary, Department of Health and Human Services), 27 January 2011 (27.01.2011), page 10, line 8 to page 11, line 6; fig. 1, 7, 8 (Family: none) | 1-4,6,7,10, 11,13,15-20 |
| Y A | WO 2008/056522 A1 (Konica Minolta Medical & Graphic, Inc.), 15 May 2008 (15.05.2008), paragraphs [0038] to [0053]; fig. 1 to 7 & JP 2008-119080 A    & JP 2008-125576 A | 1-4,6,7,10, 11,13,15-20 12 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 26 November, 2012 (26.11.12) | Date of mailing of the international search report 04 December, 2012 (04.12.12) |
| --- | --- |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2012/079005

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | WO 2011/114845 A1 (Konica Minolta Medical &<br>Graphic, Inc.),<br>22 September 2011 (22.09.2011),<br>paragraph [0170]; fig. 38<br>(Family: none) | 15,16<br>14 |
| Y | WO 2008/102685 A1 (Konica Minolta Medical &<br>Graphic, Inc.),<br>28 August 2008 (28.08.2008),<br>paragraphs [0030] to [0041], [0075] to [0077];<br>fig. 2, 3, 7<br>& US 2010/0119041 A1 | 17,19,20 |
| A | WO 2008/126787 A1 (Konica Minolta Medical &<br>Graphic, Inc.),<br>23 October 2008 (23.10.2008),<br>paragraphs [0122] to [0125], [0139]; fig. 13 to<br>16, 24<br>& JP 2010-158257 A | 5,8,9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 08102598 A
- WO 08102685 A
- JP 2011266164 A
- JP 2012236818 A

**Non-patent literature cited in the description**

- **ATSUSHI MOMOSE et al.** *Japanese Journal of Applied Physics,* October 2008, vol. 47 (10), 8077